# EUROPÄISCHE PATENTSCHRIFT

(11) **EP 4 169 608 B1**
(45) Veröffentlichungstag und Bekanntmachung des Hinweises auf die Patenterteilung: **25.02.2026**
(21) Anmeldenummer: 21204369.9
(22) Anmeldetag: 25.10.2021
(51) Int. Cl.: B01F 33/501, B01F 35/71, A61J 1/20, A61B 17/88, B01F 101/20

(54) **VORRICHTUNG ZUM BEREITSTELLEN EINER FLÜSSIGKEITSKOMPONENTE EINES KNOCHENZEMENTTEIGS, SYSTEM UND VERFAHREN ZUM BEREITSTELLEN EINES KNOCHENZEMENTTEIGS**
DEVICE FOR PROVIDING A FLUID COMPONENT OF A BONE CEMENT MASS, SYSTEM AND METHOD FOR PROVIDING A BONE CEMENT MASS
DISPOSITIF DE FOURNITURE D'UN COMPOSANT LIQUIDE D'UNE PÂTE DE CIMENT OSSEUX, SYSTÈME ET PROCÉDÉ DE FOURNITURE D'UNE PÂTE DE CIMENT OSSEUX

(43) Veröffentlichungstag der Anmeldung: 26.04.2023
(60) Teilanmeldung: 25220648.7
(73) Patentinhaber: Heraeus Medical GmbH, 61273 Wehrheim (DE)
(72) Erfinder: VOGT, Sebastian, 63450 Hanau (DE); KLUGE, Thomas, 63450 Hanau (DE)
(74) Vertreter: Heraeus IP

(56) Entgegenhaltungen:
- EP-A1- 3 150 155
- WO-A1-2005/018831
- WO-A1-2005/123162
- WO-A1-2010/145041
- US-A1- 2019 038 331
- US-B2- 8 348 494

## Beschreibung

Die Erfindung betrifft eine Vorrichtung zum Bereitstellen einer Flüssigkeitskomponente als einer ersten Ausgangskomponente eines Knochenzementteigs aus zwei Ausgangskomponenten, entsprechend Anspruch 1.

Die Erfindung betrifft weiterhin ein System zum Bereitstellen eines Knochenzementteigs aus zwei Ausgangskomponenten entsprechend Anspruch 6 sowie ein Verfahren zum Bereitstellen eines Knochenzementteigs aus zwei Ausgangskomponenten entsprechend Anspruch 12.

### Hintergrund der Erfindung

Eine häufig eingesetzte Vorgehensweise zur Behandlung von Wirbelkörperfrakturen ist die Vertebroplastie. Dabei wird ein frakturierter Wirbelkörper mit einem Knochenzement, beispielsweise einem Polymethylmethacrylat-Knochenzement oder einem anorganischen Knochenzement, stabilisiert, welcher hierzu im flüssigen bis zähflüssigen Zustand als Knochenzementteig in den Wirbelkörper eingebracht wird und dort zum Knochenzement aushärtet.

Ein Knochenzementteig wird üblicherweise durch Vermischen zweier Ausgangskomponenten, einer Flüssigkeitskomponente und einer Pulverkomponente, bereitgestellt, wobei das Aushärten des Knochenzementteigs mit dem Vermischen der Ausgangskomponenten einsetzt. Innerhalb einiger Minuten, beispielsweise innerhalb von 10 Minuten, härtet der bereitgestellte Knochenzementteig vollständig zum Knochenzement aus. Zur Durchführung der Vertebroplastie verbleibt einem Operateur nach dem Bereitstellen des Knochenzementteigs somit lediglich ein begrenztes Zeitfenster, bevor der Knochenzementteig seine Verarbeitungszeit überschritten hat und nicht mehr wie gewünscht verwendet werden kann.

Aus operationstechnischen Gründen, insbesondere aufgrund einer besseren Handhabung, werden bei der Vertebroplastie kleine Spritzen, beispielsweise mit einem Fassungsvermögen von bis zu 10 ml, verwendet. Da üblicherweise mehr Knochenzementteig als das Fassungsvermögen einer dieser kleinen Spritzen benötigt wird, müssen mehrere Spritzen mit Knochenzementteig für eine Operation bereitgestellt werden.

Die Patentanmeldung WO 2005/123162 A1 offenbart eine Vorrichtung entsprechend dem Oberbegriff des Anspruchs 1 und beschreibt eine Vorrichtung zum Befüllen einer oder mehrerer Spritzen mit frisch gemischten, injizierbaren Substanzen, umfassend ein Abgabeelement, das mit einem Hohlraum versehen ist, der eine Zuführungsöffnung zum Zuführen einer injizierbaren Mischung in den Hohlraum aufweist, und eine Vielzahl von Auslassöffnungen zum Befüllen von Spritzen, die mit dem Abgabeelement verbindbar sind.

Die Patentanmeldung WO 2005/018831 A1 beschreibt eine Umfüll-, Misch- und Abgabevorrichtung, die vorzugsweise mindestens zwei Abgabevorrichtungen, von denen eine flüssige Komponenten enthält, und eine Überführeinheit zum Verbinden der Abgabevorrichtungen aufweist.

Die Patentanmeldung WO 2010/145041 A1 beschreibt eine Befüllvorrichtung zum Brechen mindestens einer Ampulle und zum Befüllen einer Spritze aus der Ampulle offenbart. Die Befüllvorrichtung weist einen Basiskörper auf, der mindestens einen Hohlraum begrenzt. An seinem distalen Ende weist jeder der Hohlraum eine Entnahmeöffnung auf, die mit einem Anschluss für die Mehrfachspritze in Verbindung steht.

Die Patentanmeldung EP 3 150 155 A1 beschreibt eine Vorrichtung zum Mischen eines Knochenzements aufweisend zumindest eine Kartusche, wobei eine Monomerflüssigkeit als eine zweite Komponente des Knochenzements durch eine Austragsöffnung in einen Mischraum einleitbar ist.

Im Handel üblicherweise verkaufte Gebinde für Knochenzemente reichen aus, um mehrere der bei der Vertebroplastie verwendeten Spritzen zu befüllen. Ein Anmischen eines Knochenzementteigs für jeweils lediglich eine der Spritzen und verwerfen des Restes des angemischten Knochenzementteigs ist aus kommerziellen Gründen nicht sinnvoll. Daher wird bisher eine große Menge an Knochenzementteig mittels eines einzigen Gebindes, beispielsweise eines einzigen Gebindes in Form eines Behälters für die Flüssigkeitskomponente und eines einzigen Beutels enthaltend eine Pulverkomponente, bereitgestellt, welcher anschließend auf mehrere der Spritzen aufgeteilt wird.

Beispielhaft sei hier die Patentschrift US 8,348, 494 B2 genannt, in der ein zuvor bereitgestellter Knochenzementteig auf mehrere Spritzen zur Applikation in einen Patientenkörper verteilt wird. Nachteilig gestaltet sich hierbei, dass die Verarbeitungszeit des Knochenzementteigs für alle Spritzen gleichzeitig, nämlich mit dem initialen Anmischen der gesamten Menge an Knochenzementteig, zu laufen beginnt. Dies setzt den Operateur unter einen erheblichen Zeitdruck, da alle Spritzen innerhalb der Verarbeitungszeit verwendet werden müssen. Weiterhin hat dies zur Folge, dass lediglich Knochenzementteige mit einer relativ langen Verarbeitungszeit verwendet werden können, wenn mehrere mit Knochenzementteige gefüllte Spritzen zum Einsatz kommen, beispielsweise um mehrere Wirbelkörper zu stabilisieren. Dies schränkt den Operateur in seiner Auswahl an verfügbaren Knochenzementteigen ein. Gleichzeitig unterscheiden sich die rheologischen Eigenschaften des auf die Spritzen aufgeteilten initial angemischten Knochenzementteigs. Während beispielsweise der Knochenzementteig in der im Zuge der Operation zuerst verwendeten Spritze noch eine vergleichsweise geringe Viskosität aufweisen kann, wird der Knochenzementteig in der zuletzt verwendeten Spritze eine schon deutlich höhere Viskosität aufweisen. Dies erschwert die Durchführung der Operation. Zieht sich die Applikation des Knochenzementteiges zeitlich in die Länge, insbesondere über die Verarbeitungszeit des initial bereitgestellten Knochenzementteigs hinaus, kann es passieren, dass eine oder mehrere der zuvor befüllten Spritzen nicht mehr verwendet werden können. Dies macht ein erneutes Bereitstellen eines weiteren Knochenzementteigs mittels eines weiteren Gebindes, beispielsweise eines weiteren Behälters enthaltend die Flüssigkeitskomponente als Ausgangskomponente des Knochenzementteigs, notwendig, was auch aus kommerzieller Sicht nachteilig ist.

### Aufgaben

Eine Aufgabe der vorliegenden Erfindung ist es, einen oder mehrere der sich aus dem Stand der Technik ergebenen Nachteile zumindest teilweise zu überwinden.

Im Speziellen basiert die Erfindung auf dem Ziel, eine Vorrichtung zur Verfügung zu stellen, welche eine Bereitstellung von mehreren mit Knochenzementteig, bereitstellbar aus zwei Ausgangskomponenten, gefüllten Spritzen erlaubt, wobei der Knochenzementteig für alle Spritzen aus einem einzigen Gebinde umfassend ein Behältnis für eine Pulverkomponente und einen Behälter für eine Flüssigkeitskomponente, bereitstellbar sein soll ohne dass ein Anwender der Vorrichtung alle Spritzen innerhalb einer Zeitspanne verwenden muss, welche der Verarbeitungszeit des Knochenzementteigs entspricht. Die Vorrichtung soll dabei auch die Verwendung von zeitlich schnell aushärtenden Knochenzementteigen erlauben. Die Vorrichtung soll ein Anmischen des Knochenzementteigs direkt in den Spritzen ermöglich, so dass der Operateur kein Umfüllen des Knochenzementteigs in die Spritzen vornehmen muss.

Es ist eine weitere Aufgabe der Erfindung ein System zur Bereitstellung eines Knochenzementteigs aus zwei Ausgangskomponenten umfassend eine derartige Vorrichtung bereitzustellen, mittels dem mindestens ein Teil der bereits beschriebenen Aufgaben zumindest zum Teil gelöst wird.

Eine weitere Aufgabe der Erfindung besteht darin, ein Verfahren bereitzustellen, mit dem ein Knochenzementteig aus zwei Ausgangskomponenten bereitgestellt werden kann, mittels dem mindestens ein Teil der bereits beschriebenen Aufgaben zumindest zum Teil gelöst wird.

### Bevorzugte Ausführungsformen der Erfindung

Ein Beitrag zur mindestens teilweisen Erfüllung mindestens einer der zuvor genannten Aufgaben wird durch die Merkmale der unabhängigen Ansprüche geleistet. Die abhängigen Ansprüche stellen bevorzugte Ausführungsformen bereit, die zur mindestens teilweisen Erfüllung mindestens einer der Aufgaben beitragen.

### Allgemeines

In der vorliegenden Beschreibung beinhalten Bereichsangaben auch die als Grenzen genannten Werte. Eine Angabe der Art "im Bereich von X bis Y" in Bezug auf eine Größe A bedeutet folglich, dass A die Werte X, Y und Werte zwischen X und Y annehmen kann. Einseitig begrenzte Bereiche der Art "bis zu Y" für eine Größe A bedeuten entsprechend als Wert Y und kleiner als Y.

Einige der beschriebenen Merkmale sind mit dem Begriff "im Wesentlichen" verknüpft. Der Begriff "im Wesentlichen" ist so zu verstehen, dass unter realen Bedingungen und Fertigungstechniken eine mathematisch exakte Auslegung von Begrifflichkeiten wie "Überlagerung", "senkrecht", "Durchmesser" oder "Parallelität" nie exakt, sondern nur innerhalb gewisser fertigungstechnischer Fehlertoleranzen gegeben sein kann. Beispielsweise schließen "im Wesentlichen parallele Achsen" einen Winkel von 85 Grad bis 95 Grad zueinander ein und "im Wesentlichen gleiche Volumen" umfassen eine Abweichung von bis zu 5 Volumen-%. Eine "im Wesentlichen aus Kunststoff bestehende Vorrichtung" umfasst beispielsweise einen Kunststoffanteil von ≥95 bis ≤100 Gewichts-%. Eine "im Wesentlichen vollständige Befüllung eines Volumens B" umfasst beispielsweise eine Befüllung von ≥95 bis ≤100 Volumen-% des Gesamtvolumens von B.

### Ausführliche Beschreibung

Ein erster Gegenstand der Erfindung betrifft eine Vorrichtung zum Bereitstellen einer Flüssigkeitskomponente als einer ersten Ausgangskomponente eines Knochenzementteigs aus zwei Ausgangskomponenten, umfassend eine Aufnahme, in welcher ein Behälter enthaltend die Flüssigkeitskomponente lagerbar ist, ein Öffnungsmittel zum Öffnen des Behälters,
ein mit der Aufnahme fluidleitend verbundenes Reservoir zur Aufnahme der Flüssigkeitskomponente aus dem Behälter, eine Vielzahl an Anschlüssen zum fluidleitenden Verbinden der Vorrichtung mit einer Spritze, in welcher eine Pulverkomponente als eine zweite Ausgangskomponente des Knochenzementteigs lagerbar ist, und eine Vielzahl an Leitungsmitteln, wobei jeweils eines der Leitungsmittel aus der Vielzahl der Leitungsmittel das Reservoir und einen der Anschlüsse aus der Vielzahl der Anschlüsse fluidleitend miteinander verbindet,
wobei das Reservoir in eine Vielzahl an Kompartimenten unterteilt ist und wobei jeweils eines der Kompartimente über eines der Leitungsmittel mit einem der Anschlüsse fluidleitend verbunden ist,
dadurch gekennzeichnet, dass
das Reservoir mittels mindestens einer Zwischenwand in die Kompartimente unterteilt ist.

Die Vorrichtung umfasst eine Aufnahme, in der ein Behälter enthaltend die Flüssigkeitskomponente des Knochenzementteigs lagerbar ist. Unter einem Behälter werden alle Gefäße verstanden, welche die Flüssigkeitskomponente hermetisch dicht und steril lagern können und durch manuelle Krafteinwirkung zerstörbar sind. Beispiele für Behälter sind Glasampullen, Kunststoffampullen und Kunststoffbeutel. Aufgrund der leichten Sterilisierbarkeit und guten Öffenbarkeit durch manuelle Krafteinwirkung sind Glasampullen bevorzugt.

Unter eine Aufnahme wird ein Behältnis, insbesondere ein rohrartiges Behältnis, der Vorrichtung verstanden, welches zum sicheren Lagern des Behälters geeignet ist. Vorzugsweise umfasst die Aufnahme den Behälter, insbesondere die bevorzugte Glasampulle, derart, dass der Behälter sicher gegen übliche ruckartige Bewegungen, beispielsweise beim Transport der Vorrichtung, gesichert ist. Dazu kann innerhalb der Aufnahme eine Polsterung, beispielsweise aus einem Schaumstoff, angebracht sein, welche das Risiko eines ungewollten Öffnens des Behälters, beispielsweise durch ein Zerbrechen beim Transport der Vorrichtung, verringert.

Um den Behälter zu öffnen, umfasst die Vorrichtung ein Öffnungsmittel. Unter einem Öffnungsmittel ist ein Mittel zu verstehen, welches dazu geeignet ist, die strukturelle Integrität des Behälters zu zerstören und damit zu öffnen. Die Ausgestaltungsform des Öffnungsmittels ist dabei in Abhängigkeit der Art und strukturellen Stabilität des Behälters zu wählen. Ist das Behältnis beispielsweise ein Kunststoffbeutel, umfasst das Öffnungsmittel vorzugsweise ein Element, welches zum Aufschneiden, Aufstecken oder Aufreißen des Kunststoffbeutels geeignet ist, beispielsweise eine Spitze und/oder eine Schneidkante. Ist das Behältnis beispielsweise eine Glasampulle, umfasst oder ist das Öffnungsmittel beispielsweise ein Anstechdorn, eine Schneidkante, eine Brechkante.

Bevorzugt ist das Behältnis eine Glasampulle, so dass es bevorzugt ist, dass das Öffnungsmittel eine Schräge umfasst oder aus einer Schräge besteht, gegen die die Glasampulle schiebbar gelagert ist, um geöffnet zu werden. Eine Glasampulle weist üblicherweise einen Glasampullenkopf auf, welcher über einen Glasampullenhals mit einem Glasampullenkörper verbunden ist. Bevorzugt kann der Glasampullenkopf durch Schieben gegen die Schräge abgetrennt werden, so dass aus dem dadurch geöffneten Glasampullenhals die Flüssigkeitskomponente aus dem Behälter ausfließen kann.

In einer Ausgestaltungsform ist die Schräge dabei als ein separates Bauteil, angeordnet innerhalb der Vorrichtung, ausgestaltet. In einer weiteren Ausgestaltungsform ist die Schräge als Abschnitt einer Wand, insbesondere einer Innenwand, der Vorrichtung, insbesondere als Abschnitt einer Wand der Aufnahme oder einer Wand des Reservoirs, ausgestaltet. Dies reduziert die Anzahl an Bauteilen der Vorrichtung und senkt damit das Risiko einer Fehlfunktion der Vorrichtung sowie deren Herstellungskosten.

Um den Behälter, insbesondere die Glasampulle, gegen die Schräge zu schieben und so zu öffnen, kann die Vorrichtung, insbesondere die Aufnahme auf unterschiedliche Weise ausgestaltet sein. In einer Ausgestaltungsform ist die Aufnahme zumindest abschnittsweise in die Vorrichtung hineinschiebbar, um den Behälter gegen die Schräge zu schieben. Beispielsweise kann die Aufnahme einen hinteren Aufnahmeabschnitt umfassen, welcher in einen, dem Öffnungsmittel zugewandten, vorderen Aufnahmeabschnitt zumindest abschnittsweise einschiebbar ist, um den Behälter zu öffnen. In einer weiteren Ausgestaltungsform ist die Aufnahme oder zumindest ein Teil der Aufnahme knickbar, um den Behälter gegen das Öffnungsmittel, insbesondere in Form der Schräge, mittels einer Rotationsbewegung gegen das Öffnungsmittel, insbesondere in Form der Schräge, zu schieben und so zu öffnen. Um ein unbeabsichtigtes Öffnen des Behälters zu verhindern, kann die Vorrichtung mit einer Transportsicherung ausgestaltet sein, welches ein Einschieben der Aufnahme oder ein Abknicken der Aufnahme verhindert, bis die Transportsicherung vor dem Verwenden der Vorrichtung durch einen Anwender der Vorrichtung entfernt wird.

Um ein Ausfließen der Flüssigkeitskomponente aus dem Behälter, insbesondere eines Behälters in Form einer Glasampulle, nach dessen Öffnung zu erleichtern, ist die Aufnahme vorzugsweise so ausgestaltet, dass der Behälter bei einem ordnungsgemäßen Aufstellen der Vorrichtung auf einer waagrechten Oberfläche, beispielsweise einem Tisch, einen Winkel zu einer Senkrechten der Oberfläche in einem Bereich von 10° bis 30°, bevorzugt von 15° bis 25°, einnimmt.

Die Aufnahme der Vorrichtung ist fluidleitend mit einem Reservoir verbunden. Dabei können die Aufnahme und das Reservoir unmittelbar fluidleitend zueinander ausgestaltet sein, oder zwischen der Aufnahme und dem Reservoir ist ein fluidleitendes Element, beispielsweise ein Rohr oder ein Schlauch angeordnet, welches eine fluidleitende Verbindung herstellt. Das Reservoir dient der kontaminationsfreien, im Wesentlichen sterilen, Zwischenlagerung der Flüssigkeitskomponente, welche nach dem Öffnen des Behälters aus diesem herausfließt, bis zu einem Anmischen mit einer Pulverkomponente als zweiter Ausgangskomponente des Knochenzementteigs. Das Reservoir lagert die Flüssigkeitskomponente kontaminationsfrei, und bei der Verwendung von Methymethacrylat als Flüssigkeitskomponente auch im Wesentlichen ohne Geruchsbelästigung für den Anwender der Vorrichtung, derart, dass die Flüssigkeitskomponente einfach, schnell und im Volumen bedarfsgereicht entweder im Ganzen oder in mehreren, portionsweisen Entnahmevorgängen zur Anmischung des Knochenzementteigs entnehmbar ist. Dies erlaubt ein zeitversetztes Anmischen mehrerer Portionen an Knochenzementteig. Dadurch ist es möglich, immer nur eine derzeit notwendige Menge an Knochenzementteig bereitzustellen, diese zu verwenden und erst danach eine weitere Portion Knochenzementteig aus dem gleichen Behälter der Flüssigkeitskomponent anzumischen. Somit steht dem Operateur für jede dieser Portionen des Knochenzementteigs die gesamte Verarbeitungszeit des verwendeten Knochenzementteigs zur Verfügung, was bei einer initialen Anmischung des gesamten Behälters nicht möglich wäre.

Dabei ist das Reservoir vorzugsweise so dimensioniert, dass die Flüssigkeitskomponente vollständig innerhalb des Reservoirs gelagert werden kann. Beispielsweise fasst das Reservoir ein Volumen von bis zu 50 ml einer Flüssigkeit.

Zur Aufnahme der Flüssigkeitskomponente kann das Reservoir auf unterschiedliche Weise ausgestaltet sein. In einer Ausgestaltungsform ist das Reservoir schalenartig ausgestaltet, um die Komplexität der Vorrichtung weitgehend zu reduzieren. Bevorzugt umfasst das Reservoir dabei eine der Aufnahme zugewandte fluidleitend geöffnete Reservoiröffnung Dies erlaubt ein einfaches Einfließen der Flüssigkeitskomponente aus dem Behälter in das schalenartige Reservoir über die der Aufnahme zugewandte Reservoiröffnung.

Vorzugsweise liegt das Reservoir bei ordnungsgemäßer Aufstellung der Vorrichtung auf einer waagrechten Oberfläche räumlich tiefer, also näher an der waagrechten Oberfläche, als die Aufnahme, so dass die Flüssigkeitskomponente ohne Zutun eines Anwenders gemäß der Schwerkraft aus dem Behälter in das Reservoir fließbar ist.

Um zu verhindern, dass der Behälter oder Behälterfragmente nach dem Öffnen des Behälters, insbesondere Glasfragmente nach dem Öffnen eines Behälters in Form einer Glasampulle, in das Reservoir gelangen, kann zwischen der Aufnahme und dem Reservoir ein Rückhalteelement angeordnet sein. Das Rückhalteelement kann beispielsweise ein Sieb, eine offenporöse Platte, insbesondere eine offenporöse Kunststoffplatte, oder einen offenporösen Stift, insbesondere einen offenporösen Kunststoffstift, umfassen oder aus dem Vorgenannten bestehen.

Um die Flüssigkeitskomponente aus dem Reservoir in eine Spritze zur weiteren Verwendung zu fördern, umfasst die Vorrichtung mindestens einen Anschluss, welcher fluidleitend über ein Leitungsmittel mit dem Reservoir verbunden ist.

Das Leitungsmittel kann unterschiedlich ausgeformt sein, um den Anschluss und das Reservoir fluidleitend zu verbinden. Beispielsweise kann das Leitungsmittel als Kanal ausgeformt sein. Vorzugsweise ist das Leitungsmittel derart mit dem Reservoir verbunden, dass bei einem ordnungsgemäßen Aufstellen der Vorrichtung im Wesentlichen die gesamte im Reservoir vorhandene Flüssigkeitskomponente durch das Leitungsmittel in Richtung des Anschlusses gefördert werden kann. Beispielsweise ist das Leitungsmittel im Bereich des bei einem ordnungsgemäßen Aufstellen der Vorrichtung räumlich tieftesten Punktes des Reservoirs angeordnet.

Über den Anschluss kann eine Spritze reversible fluidleitend mit dem Reservoir verbunden werden, um die im Reservoir zwischengelagerte Flüssigkeitskomponente in die Spritze zu fördern. Um die fluidleitende Verbindung von Vorrichtung und Spritze herzustellen, kann der Anschluss auf unterschiedliche Weisen ausgestaltet sein. In einer Ausgestaltungsform ist der Anschluss als Gewinde ausgeformt, um mit einem entsprechenden Gegenstück an der Spritze die fluidleitende Verbindung herzustellen. Beispielsweise kann der Anschluss ein Innengewinde aufweisen, welches mit einem Außengewinde an einer Spritze die fluidleitende Verbindung ausformt. In einer weiteren Ausgestaltungsform formen der Anschluss und die Spritze eine Bajonettverbindung aus. In einer weiteren Ausgestaltungsform können der Anschluss und die Spritze durch teilweises Einschieben der Spritze in den Anschluss fluidleitend miteinander verbunden werden. Beispielsweise kann das Leitungsmittel als Schlauch ausgeformt sein, um das Reservoir und den Anschluss fluidleitend zu verbinden, und eine Spritze kann am Anschluss über ein abschnittsweises Einschieben der Spritze in den Schlauch die fluidleitende Verbindung von Anschluss und Spritze ausformen. Nach dem Fördern der Flüssigkeitskomponente kann die Spritze vom Anschluss zur weiteren Verwendung gelöst werden.

Um ein Eindringen von Behälterfragmenten, insbesondere von Glasfragmente entstanden durch Öffnen eines Behälters in Form einer Glasampulle, in eine mit dem Anschluss fluidleitend verbundene Spritze zu unterbinden, kann der Anschluss mit einer Filtereinheit ausgestattet sein. Die Filtereinheit kann beispielsweise ein Sieb, eine offenporöse Platte, insbesondere eine offenporöse Kunststoffplatte, oder einen offenporösen Stift, insbesondere einen offenporösen Kunststoffstift, umfassen oder aus dem Vorgenannten bestehen. In einer Ausgestaltungsform sind der Anschluss und die Filtereinheit irreversibel miteinander verbunden, beispielsweise über eine Klebeverbindung. In einer weiteren Ausgestaltungsform sind der Anschluss und die Filtereinheit reversibel miteinander verbunden, beispielsweise ist die Filtereinheit als ein mit dem Anschluss verbindbarer Adapter ausgeformt. Dabei kann der Adapter beispielsweise über eine Gewindeverbindung oder eine Bajonettverbindung mit dem Anschluss verbunden werden. Der Adapter kann beispielsweise wiederum mit einer dem Anschluss abgewandten Seite fluidleitend mit der Spritze verbunden werden, so dass der Anschluss über den Adapter fluidleitend mit der Spritze verbunden ist.

Um ein versehentliches Fließen der Flüssigkeitskomponente über den mindestens einen Anschluss in eine mit dem mindestens einen Anschluss fluidleitend verbundene Spritze zu verhindern, ist es bevorzugt, dass, bei ordnungsgemäßer Aufstellung der Vorrichtung auf einer waagrechten Oberfläche, das Reservoir räumlich tiefer, also näher an der waagrechten Oberfläche, liegt als der mindestens eine Anschluss.

Die Vorrichtung ist dadurch gekennzeichnet, dass die Vorrichtung eine Vielzahl an Anschlüssen, insbesondere zwei, drei, vier oder fünf Anschlüsse, zum fluidleitenden Verbinden der Vorrichtung mit einer Spritze, in welcher eine Pulverkomponente als zweite Ausgangskomponente des Knochenzementteigs lagerbar ist, und eine Vielzahl an Leitungsmitteln, insbesondere zwei, drei, vier oder fünf Leitungsmittel, umfasst, wobei jeweils eines der Leitungsmittel aus der Vielzahl der Leitungsmittel das Reservoir und einen der Anschlüsse aus der Vielzahl der Anschlüsse fluidleitend miteinander verbindet. Die Vorrichtung umfasst eine Vielzahl an Leitungsmittel-Anschluss-Paarungen, um die Vorrichtung fluidleitend mit einer Vielzahl an Spritzen gleichzeitig fluidleitend verbinden zu können. Durch die Vielzahl an Anschlüssen kann die Vorrichtung bereits vor dem ersten Anmischen einer Portion Knochenzementteig mit einer Vielzahl an Spritzen fluidleitend verbunden werden, was einem Anwender der Vorrichtung eine Verwendung mehrerer Spritzen im Zuge der Operation erleichtert. Zudem kann für den Fall einer Verstopfung eines der Anschlüsse auf einen anderen Anschluss zurückgegriffen werden.

Die Vorrichtung ist dadurch gekennzeichnet, dass das Reservoir in eine Vielzahl an Kompartimenten unterteilt ist, wobei jeweils eines der Kompartimente über eines der Leitungsmittel mit einem der Anschlüsse fluidleitend verbunden ist. Durch die Kompartimente ist das Reservoir in kleinere Unterreservoire unterteilt, was eine Vorportionierung der aus dem Behälter ausgeflossenen Flüssigkeitskomponente innerhalb des Reservoirs erlaubt. Da die einzelnen Kompartimente über jeweils ein separates Leitungsmittel mit einem separaten Anschluss fluidleitend verbunden sind, kann über jeden dieser separaten Anschlüsse eine vorportionierte Flüssigkeitskomponentenmenge aus der Vorrichtung entnommen werden. So wird sichergestellt, dass immer nur eine gewünschte, vordefinierte Menge an Flüssigkeitskomponente aus einem der Anschlüsse entnehmbar ist, was dem Anwender der Vorrichtung die Anwendung erleichtert.

Die Vorrichtung ist dadurch gekennzeichnet, dass das Reservoir mittels mindestens einer Zwischenwand in die Kompartimente unterteilt ist. Beispielsweise kann eine einzelne Zwischenwand das Reservoir in zwei Kompartimente oder zwei sich kreuzende Zwischenwände das Reservoir in vier Kompartimente unterteilen. Zwischenwände erlauben eine einfache und kostengünstige Konstruktionsweise des in Kompartimente unterteilten Reservoirs.

In einer Ausführungsform weisen die einzelnen Kompartimente unterschiedliche Volumina auf, so dass aus den entsprechenden Anschlüssen der Vorrichtung unterschiedliche Mengen der Flüssigkeitskomponente entnehmbar sind. Dies erlaubt die zeitlich gestaffelte Anmischung unterschiedlicher Portionen an Knochenzementteig aus einem Behälter enthaltend die Flüssigkeitskomponente. Die Portionen können sich beispielsweise Volumen und/oder Viskosität unterschieden.

Eine Ausführungsform der Vorrichtung ist dadurch gekennzeichnet, dass die einzelnen Kompartimente jeweils ein im Wesentlichen gleichgroßes Volumen aufweisen. Dies erlaubt die zeitlich gestaffelte Anmischung im Wesentlichen gleichartiger Portionen an Knochenzementteig mittels eines einzigen Behälters der Flüssigkeitskomponente. Die Portionen können sich beispielsweise, bei gleichzeitiger Verwendung gleicher Teil an Pulverkomponente, in ihren Volumina und Viskositäten gleichen.

Eine Ausführungsform der Vorrichtung ist dadurch gekennzeichnet, dass die Kompartimente jeweils an einer der Aufnahme zugewandten oberen Kompartimentseite fluidleitend geöffnet sind, so dass die Kompartimente untereinander über die oberen Kompartimentseiten fluidleitend miteinander verbunden sind. Beispielsweise kann eine erste Zwischenwand das Reservoir, insbesondere ein schalenartiges Reservoir, welches eine der Aufnahme zugewandten fluidleitend geöffnete Reservoirseite aufweist, in zwei Kompartimente unterteilen, welche jeweils eine der Aufnahme zugewandte fluidleitend geöffnete obere Kompartimentseite aufweisen. Weiterhin kann das Reservoir beispielsweise durch eine zweite, die erste Zwischenwand kreuzende, Zwischenwand in vier Kompartimente mit entsprechend vier fluidleitend geöffneten oberen Kompartimentseiten unterteilt werden.

Das Leitungsmittel kann unterschiedlich ausgeformt sein, um das Reservoir oder die einzelnen Kompartimente des Reservoirs mit dem Anschluss oder den Anschlüssen fluidleitend zu verbinden.

Eine Ausführungsform der Vorrichtung ist dadurch gekennzeichnet, dass das mindestens eine Leitungsmittel ein Schlauch ist. Ein Schlauch erlaubt eine einfache, günstige und flexible fluidleitende Verbindung von Reservoir und Anschluss.

Eine Ausführungsform der Vorrichtung ist dadurch gekennzeichnet, dass der Schlauch einen Innendurchmesser in einem Bereich von 0,5 mm bis 3 mm, bevorzugt zwischen 0,5 mm und 2,5 mm, weiter bevorzugt zwischen 0,5 mm und 2 mm aufweist. Aufgrund der Oberflächenspannung der Flüssigkeitskomponente verhindert ein derartiger Innendurchmesser des Schlauchs ein selbstständiges Abfließen der Flüssigkeitskomponente aus dem Reservoir durch das Leitungsmittel in Richtung des Anschlusses. Die Flüssigkeitskomponente verbleibt mit einem derartigen Innendurchmesser des Schlauchs innerhalb des Reservoirs, bis der Anwender aktiv ein Fördern aus dem Reservoir in Richtung des Anschlusses vorantreibt, beispielsweise durch Ausübung eines Förderdrucks auf die Flüssigkeitskomponente oder durch Anlegen eines Unterdrucks durch das Leitungsmittel aus Richtung des Anschlusses, beispielsweise ausgeübt durch eine Betätigung einer mit dem Anschluss fluidleitend verbundener Spritze.

Ein weiterer Gegenstand der Erfindung betrifft ein System zum Bereitstellen eines Knochenzementteigs aus zwei Ausgangskomponenten umfassend eine Vorrichtung nach einer der vorhergehenden Ausführungsformen, wobei in der Aufnahme ein Behälter, bevorzugt ein Behälter in der Form einer Glasampulle, enthaltend eine Flüssigkeitskomponente als erste Ausgangskomponente lagert und wobei das System eine Vielzahl an Spritze, insbesondere zwei, drei, vier oder fünf Spritzen, umfasst, wobei die Spritzen jeweils eine Pulverkomponente als zweite Ausgangskomponente enthalten.

Unter einem Behälter werden alle Gefäße verstanden, welche die Flüssigkeitskomponente hermetisch dicht und steril lagern können und durch manuelle Krafteinwirkung zerstörbar sind. Beispiele für Behälter sind Glasampullen, Kunststoffampullen und Kunststoffbeutel. Aufgrund der leichten Sterilisierbarkeit und guten Öffenbarkeit durch manuelle Krafteinwirkung sind Glasampullen bevorzugt.

Das System umfasst eine Vielzahl an Spritzen, welche eine Pulverkomponente als zweite Ausgangskomponente des Knochenzementteigs enthalten. Dies erlaubt ein einfaches und schnelles Anmischen des Knochenzementteigs nach dem Fördern der Flüssigkeitskomponente aus dem Behälter über das Reservoir, das Leitungsmittel und den Anschluss in eine der Spritzen direkt in dieser Spritze. Ein Umfüllen des Knochenzementteigs in eine der Spritzen entfällt dadurch. Zum Anmischen des Knochenzementteigs in der Spritze kann diese eine Mischeinrichtung, wie beispielsweise einen Mischstab oder eine oder mehrere Mischkugeln enthalten. Eine Mischkugel dient einem Anmischen des Knochenzementteigs durch Schütteln der Spritze, indem die Mischkugel mitsamt den beiden Ausgangskomponenten durch das Schütteln in der Spritze hin- und herbewegt und dadurch das Anmischen unterstützt wird.

In einer Ausführungsform des Systems sind die Spritzen mischeinrichtungsfrei ausgestaltet.

Unter einer Spritze ist ein Behältnis zu verstehen, welches die Pulverkomponente kontaminationsfrei lagern kann und mittels dem ein Knochenzementteig an eine gewünschte Stelle appliziert werden kann. Bevorzugt umfasst die Spritze einen Spritzenkolben, welcher durch Vortreiben in Richtung einer Austragsöffnung der Spritze den Knochenzementteig aus der Spritze austragen kann. Zum Austragen des Knochenzementteigs kann an der Spritze, insbesondere an der Austragsöffnung, eine Austragshilfe, wie beispielsweise eine Kanüle, ein Austragsschnorchel oder ein Schlauch, befestigt werden.

Die Spritze ist reversibel fluidleitend mit dem Anschluss der Vorrichtung verbindbar. Dazu kann die Spritze beispielsweise ein Gewinde, insbesondere ein Außengewinde, oder einen Teil einer Bajonettverbindung umfassen.

Um zu verhindern, dass mit der Flüssigkeitskomponente Fragmente des Behälters in die Spritze transferiert werden, kann die Spritze einen Filter umfassen. Der Filter kann beispielsweise ein Sieb, eine offenporöse Platte, insbesondere eine offenporöse Kunststoffplatte, oder einen offenporösen Stift, insbesondere einen offenporösen Kunststoffstift, umfassen oder aus dem Vorgenannten bestehen. In einer Ausgestaltungsform sind die Spritze und der Filter reversibel miteinander verbunden, beispielsweise ist Filter als ein mit der Spritze verbindbarer Spritzenadapter ausgeformt. Dabei kann der Spritzenadapter beispielsweise über eine Gewindeverbindung oder eine Bajonettverbindung mit der Spritze verbunden werden. Vor dem Applizieren des Knochenzementteigs aus der Spritze heraus wird der Filter vorzugweise von der Spritze, beispielsweise durch Abschrauben, entfernt. In einer weiteren Ausgestaltungsform ist der Filter im Bereich der Austragsöffnung in die Spritze eingeschoben und wird beim Austragen des Knochenzementteigs durch diesen aus der Spritze, insbesondere aus der Austragsöffnung, ausgeschoben.

Das System ist vorzugsweise so ausgelegt, dass die enthaltene Flüssigkeitskomponente für ein Anmischen eines applizierbaren und für Operationen verwendbaren Knochenzementteigs in mehreren, vorzugsweise in allen, Spritzen des Systems ausreicht. Beispielsweise enthält der Behälter 30 ml Flüssigkeitskomponente und die Vielzahl an Spritzen enthält insgesamt 45 g Pulverkomponente.

Das System erlaubt ein zeitversetztes Anmischen mehrerer Portionen Knochenzementteig aus einem einzigen Gebinde, insbesondere mittels eines einzigen Behälters der Flüssigkeitskomponente. Dadurch wird der Zeitdruck auf einen Operateur reduziert, welcher ansonsten den gesamten initial angemischten Knochenzementteig innerhalb der Verarbeitungszeit desselben verwenden müsste. Die zeitversetzte, portionsweise Anmischung startet für jede der Spritzen eine separate Verarbeitungszeit, so dass insgesamt eine längere Zeitspanne für die Verwendung des Knochenzementteigs zur Verfügung steht.

Um ein versehentliches Fließen der Flüssigkeitskomponente über den mindestens einen Anschluss in eine mit dem mindestens einen Anschluss fluidleitend verbundene Spritze zu verhindern, ist es bevorzugt, dass, bei ordnungsgemäßer Aufstellung der Vorrichtung auf einer waagrechten Oberfläche, das Reservoir räumlich tiefer, also näher an der waagrechten Oberfläche, liegt als der mindestens eine Anschluss. Zudem ist es bevorzugt, dass die Flüssigkeitskomponente nach dem Fließen in das Reservoir bei ordnungsgemäßer Aufstellung der Vorrichtung auf einer waagrechten Oberfläche einen Flüssigkeitsspiegel aufweist, welcher räumlich tiefer liegt als der mindestens eine Anschluss.

Eine Ausführungsform des Systems ist dadurch gekennzeichnet, dass die Vorrichtung eine Vielzahl an Anschlüssen, insbesondere zwei, drei, vier oder fünf Anschlüsse, aufweist. Dabei ist es bevorzugt, dass das System genauso viele Spritzen wie Anschlüsse aufweist. Beispielsweise weist das System zwei Anschlüsse und zwei Spritzen auf.

Dies erlaubt ein gleichzeitig fluidleitendes Verbinden aller Spritzen mit der Vorrichtung bereits vor dem Beginn einer Operation, was den Operationsablauf für den Operateur erleichtert.

Eine Ausführungsform des Systems ist dadurch gekennzeichnet, dass die Spritzen reversibel fluidleitend mit den Anschlüssen verbunden sind. Dies verschließt sowohl die Vorrichtung als auch die Spritzen kontaminationsfrei, so dass das System, insbesondere der Behälter und die Pulverkomponente innerhalb des Systems, steril lagerbar ist. Insbesondere kann das derartige System aus einer sterilen Packung entnommen werden, ohne dass dies ein Kontaminationsrisiko für das System, insbesondere des Behälters und der Pulverkomponente innerhalb des Systems, darstellt. Zudem verringern sich dadurch mögliche Anwendungsfehler des Systems durch einen Operateur.

Eine Ausführungsform des Systems, wobei die Kompartimente der Vorrichtung des Systems jeweils an einer der Aufnahme zugewandten oberen Kompartimentseite fluidleitend geöffnet sind, so dass die Kompartimente über die oberen Kompartimentseiten fluidleitend miteinander verbunden sind und wobei jeweils eines der Kompartimente über eines der Leitungsmittel mit einem der Anschlüsse der Vorrichtung fluidleitend verbunden ist, ist dadurch gekennzeichnet, dass im Behälter eine derartige Menge an Flüssigkeitskomponente lagert, dass nach einem Öffnen des Behälters alle Kompartimente des Reservoirs mit der Flüssigkeitskomponente befüllbar sind und dabei jeweils eine Füllhöhe aufweisen, welche eine Höhe der die jeweiligen Kompartimente trennenden mindestens einen Zwischenwand zumindest abschnittsweise überragt.

Dies erlaubt ein Befüllen aller Kompartimente mit der Flüssigkeitskomponente, da sich die aus dem Behälter in das Reservoir einfließende Flüssigkeitskomponente über die oberen Kompartimentseiten über die jeweils trennende Zwischenwand hinweg verteilt.

Beispielsweise fließt die Flüssigkeitskomponente aus dem Behälter lediglich in eines der Kompartimente des Reservoirs und füllt dieses. Ist das Volumen dieses Kompartiments gefüllt, wird weitere, in dieses Kompartiment fließende Flüssigkeitskomponente aus dem bereits gefüllten Kompartiment über die Zwischenwand des Kompartiments in ein angrenzendes Kompartiment überfließen. Auf diese Weise kann sich die Monomerflüssigkeit nacheinander in alle Kompartimente des Reservoirs verteilen und diese befüllen. Die Menge an Flüssigkeitskomponente ist dabei so abgestimmt, dass ein vollständiges Befüllen aller Kompartimente möglich ist und die Flüssigkeitskomponente jeweils einen Füllstand in den Kompartimenten aufweist, welcher zumindest abschnittsweise höher ist als die entsprechende Zwischenwand. Dies erlaubt ein "Angleichen" der Füllhöhe der Monomerflüssigkeit über alle Kompartimente hinweg. Die Kompartimente weisen somit einen Überstand auf, welcher die zumindest eine Zwischenwand überragt.

Eine Ausführungsform des Systems ist dadurch gekennzeichnet, dass die Füllhöhe der Kompartimente zumindest abschnittsweise die Höhe der zumindest einen Zwischenwand um nicht mehr als 1 mm überragt. In anderen Worten ist der Überstand nicht höher als 1 mm. Dies erlaubt ein gutes Verteilen der Flüssigkeitskomponente in alle Kompartimente. Der Überstand fließt mit dem Entleeren des ersten Kompartiments in dieses hinein und wird mitentleert. Dies sorgt beispielsweise dafür, dass trotz gleich großer Kompartimente das zuerst geleerte Kompartiment den größten Anteil an Flüssigkeitskomponente liefert. Durch den geringen Überstand von maximal 1 mm wird sichergestellt, dass die Kompartimente unabhängig von der Reihenfolge ihrer Entleerung im Wesentlichen das durch die Kompartimente vordefinierte Volumen an Flüssigkeitskomponente bereitstellen.

Eine Ausführungsform des Systems ist dadurch gekennzeichnet, dass in den Spritzen eine im Wesentlichen gleiche Menge der Pulverkomponente gelagert ist. Dies verhindert Verwechslungsgefahren der einzelnen Spritzen, welche unter Umständen Knochenzemente mit unterschiedlichen Eigenschaften, wie beispielsweise ihrer Rheologie, liefern würden. Bevorzugt weisen zudem die Kompartimente im Wesentlichen gleich große Volumina auf, so dass mit jeder Spritze ein Knochenzementteig mit im Wesentlichen gleichen Eigenschaften, insbesondere im Wesentlichen gleicher Rheologie, erhältlich ist.

Ein weiterer Gegenstand der Erfindung betrifft ein Verfahren entsprechend Anspruch 12 zum Bereitstellen eines Knochenzementteigs aus zwei Ausgangskomponenten mittels eines Systems, vorzugsweise mittels eines Systems nach einer der vorhergehenden Ausführungsformen, wobei die Vielzahl an Spritzen zumindest eine erste Spritze und eine zweite Spritze umfasst, wobei das System eine Vorrichtung, vorzugsweise eine Vorrichtung nach einer der vorhergehenden Ausführungsformen, umfasst, aufweisend die folgenden Schritte:
a. Öffnen eines Behälters mittels eines Öffnungsmittels,
b. Fließen der Flüssigkeitskomponente aus dem geöffneten Behälter in ein Reservoir,
c. Fördern eines ersten Teils der Flüssigkeitskomponente aus dem Reservoir in die erste Spritze,
d. Fördern eines zweiten Teils der Flüssigkeitskomponente aus dem Reservoir in die zweite Spritze.

Das Öffnen des Behälters kann auf unterschiedliche Weise vonstattengehen, wobei es bevorzugt ist, dass der Behälter eine Glasampulle ist und das Öffnen der Glasampulle durch ein Schieben oder eine Rotationsbewegung der Glasampulle gegen das Öffnungsmittel, vorzugsweise gegen das Öffnungsmittel in Form einer Schräge, geschieht. Dabei wird vorzugsweise ein Glasampullenkopf abgetrennt, was den Behälter öffnet.

Nach dem Öffnen des Behälters fließt die Flüssigkeitskomponente aus dem geöffneten Behälter in das Reservoir, bevorzugt das schalenförmige Reservoir. Vorzugsweise geschieht das getrieben durch die Schwerkraft ohne aktives Zutun eines Anwenders des Systems des Verfahrens.

Um eine erste Portion des Knochenzementteigs bereitstellen zu können, wird der erste Teil der Flüssigkeitskomponente aus dem Reservoir in die erste Spritze gefördert. Der zweite Teil der Flüssigkeitskomponente verbleibt im Reservoir.

Zeitversetzt zum Fördern des ersten Teils der Flüssigkeitskomponente wird der zweite Teil der Flüssigkeitskomponente in die zweite Spritze gefördert. Ein weiterer Teil der Flüssigkeitskomponente kann dabei weiterhin im Reservoir verbleiben und für ein Fördern in eine weitere sowie für ein zusätzliches Fördern in die erste oder zweite Spritze zur Verfügung stehen.

Das Fördern der Flüssigkeitskomponenten in die erste Spritze und die zweite Spritze kann auf unterschiedliche Weisen durchgeführt werden. In einer bevorzugten Ausführungsform des Verfahrens geschieht das Fördern jeweils durch einen Kolbenhub eines zu den Spritzen zugehörigen Spritzenkolbens. Dadurch kann das Fördern ohne separate Werkzeuge oder Gerätschaften, wie beispielsweise eine Pumpe durchgeführt werden, was das Verfahren erleichtert.

Eine Ausführungsform des Verfahrens, wobei das System des Verfahrens einen ersten Anschluss und ein erstes Leitungsmittel zum fluidleitenden Verbinden des ersten Anschlusses mit dem Reservoir sowie einen zweiten Anschluss und ein zweites Leitungsmittel zum fluidleitenden Verbinden des zweiten Anschlusses mit dem Reservoir aufweist, ist dadurch gekennzeichnet, dass das Fördern des ersten Teils der Flüssigkeitskomponente aus dem Reservoir in die erste Spritze über den ersten Anschluss und das Fördern des zweiten Teils der Flüssigkeitskomponente aus dem Reservoir in die zweite Spritze über den zweiten Anschluss erfolgt. Weitere Teile der Flüssigkeitskomponente können über weitere Anschlüsse, welche über weitere Leitungsmittel fluidleitend mit dem Reservoir verbunden sind, in weitere oder die gleichen Spritzen gefördert werden.

Auf diese Weise können die erste Spritze und die zweite Spritze gleichzeitig mit der Vorrichtung des Verfahrens fluidleitend verbunden werden, beispielsweise bereits vor dem Beginn einer Operation, was die Anwendung des Verfahrens erleichtert. Zudem kann des Systems des Verfahrens bereits mit fluidleitenden Spritzen steril verpackt werden, was das Kontaminationsrisiko des Systems des Verfahrens nach dem Auspacken aus der sterilen Verpackung verringert.

Eine Ausführungsform des Verfahrens, wobei das Reservoir ein erstes Kompartiment und ein zweites Kompartiment aufweist, ist dadurch gekennzeichnet, dass nach dem Öffnen des Behälters der erste Teil der Flüssigkeitskomponente in das erste Kompartiment und der zweite Teil der Flüssigkeitskomponente in das zweite Kompartiment fließt, wobei das erste Kompartiment über das erste Leitungsmittel mit dem ersten Anschluss und das zweite Kompartiment über das zweite Leitungsmittel mit dem zweiten Anschluss fluidleitend verbunden ist und das Fördern des ersten Teils der Flüssigkeitskomponente aus dem ersten Kompartiment in die erste Spritze und das Fördern des zweiten Teils der Flüssigkeitskomponente aus dem zweiten Kompartiment in die zweite Spritze erfolgt. Durch das erste Kompartiment und das zweite Kompartiment, und optional weiterer Kompartimente, wird die Flüssigkeitskomponente im Reservoir vorportioniert, so dass über die Anschlüsse lediglich eine im Wesentlichen vordefinierte Menge an Flüssigkeitskomponente aus der Vorrichtung des Verfahrens entnehmbar ist. Ein Anwender des Systems des Verfahrens muss daher nicht darauf achten, wieviel der Flüssigkeitskomponente in die jeweilige Spritze gefördert wird, was das Risiko einer falsch dosierten Entnahme an Flüssigkeitskomponente verringert.

Die Vorrichtung des Verfahrens umfasst eine Aufnahme, in der ein Behälter enthaltend die Flüssigkeitskomponente des Knochenzementteigs lagerbar ist. Unter einem Behälter werden alle Gefäße verstanden, welche die Flüssigkeitskomponente hermetisch dicht und steril lagern können und durch manuelle Krafteinwirkung zerstörbar sind. Beispiele für Behälter sind Glasampullen, Kunststoffampullen und Kunststoffbeutel. Aufgrund der leichten Sterilisierbarkeit und guten Öffenbarkeit durch manuelle Krafteinwirkung sind Glasampullen bevorzugt.

Unter eine Aufnahme wird ein Behältnis, insbesondere ein rohrartiges Behältnis, der Vorrichtung des Verfahrens verstanden, welches zum sicheren Lagern des Behälters geeignet ist. Vorzugsweise umfasst die Aufnahme den Behälter, insbesondere die bevorzugte Glasampulle, derart, dass der Behälter sicher gegen übliche ruckartige Bewegungen, beispielsweise beim Transport der Vorrichtung des Verfahrens, gesichert ist. Dazu kann innerhalb der Aufnahme eine Polsterung, beispielsweise aus einem Schaumstoff, angebracht sein, welche das Risiko eines ungewollten Öffnens des Behälters, beispielsweise durch ein Zerbrechen beim Transport der Vorrichtung des Verfahrens, verringert.

Um den Behälter zu öffnen, umfasst die Vorrichtung des Verfahrens ein Öffnungsmittel. Unter einem Öffnungsmittel ist ein Mittel zu verstehen, welches dazu geeignet ist, die strukturelle Integrität des Behälters zu zerstören und damit zu öffnen. Die Ausgestaltungsform des Öffnungsmittels ist dabei in Abhängigkeit der Art und strukturellen Stabilität des Behälters zu wählen. Ist das Behältnis beispielsweise ein Kunststoffbeutel, umfasst das Öffnungsmittel vorzugsweise ein Element, welches zum Aufschneiden, Aufstecken oder Aufreißen des Kunststoffbeutels geeignet ist, beispielsweise eine Spitze und/oder eine Schneidkante. Ist das Behältnis beispielsweise eine Glasampulle, umfasst oder ist das Öffnungsmittel beispielsweise ein Anstechdorn, eine Schneidkante, eine Brechkante.

Bevorzugt ist das Behältnis eine Glasampulle, so dass es bevorzugt ist, dass das Öffnungsmittel eine Schräge umfasst oder aus einer Schräge besteht, gegen die die Glasampulle schiebbar gelagert ist, um geöffnet zu werden. Eine Glasampulle weist üblicherweise einen Glasampullenkopf auf, welcher über einen Glasampullenhals mit einem Glasampullenkörper verbunden ist. Bevorzugt kann der Glasampullenkopf durch Schieben gegen die Schräge abgetrennt werden, so dass aus dem dadurch geöffneten Glasampullenhals die Flüssigkeitskomponente aus dem Behälter ausfließen kann.

In einer Ausgestaltungsform ist die Schräge dabei als ein separates Bauteil, angeordnet innerhalb der Vorrichtung des Verfahrens, ausgestaltet. In einer weiteren Ausgestaltungsform ist die Schräge als Abschnitt einer Wand, insbesondere einer Innenwand, der Vorrichtung des Verfahrens, insbesondere als Abschnitt einer Wand der Aufnahme oder einer Wand des Reservoirs, ausgestaltet. Dies reduziert die Anzahl an Bauteilen der Vorrichtung des Verfahrens und senkt damit das Risiko einer Fehlfunktion der Vorrichtung des Verfahrens sowie deren Herstellungskosten.

Um den Behälter, insbesondere die Glasampulle, gegen die Schräge zu schieben und so zu öffnen, kann die Vorrichtung des Verfahrens, insbesondere die Aufnahme auf unterschiedliche Weise ausgestaltet sein. In einer Ausgestaltungsform ist die Aufnahme zumindest abschnittsweise in die Vorrichtung des Verfahrens hineinschiebbar, um den Behälter gegen die Schräge zu schieben. Beispielsweise kann die Aufnahme einen hinteren Aufnahmeabschnitt umfassen, welcher in einen, dem Öffnungsmittel zugewandten, vorderen Aufnahmeabschnitt zumindest abschnittsweise einschiebbar ist, um den Behälter zu öffnen. In einer weiteren Ausgestaltungsform ist die Aufnahme oder zumindest ein Teil der Aufnahme knickbar, um den Behälter gegen das Öffnungsmittel, insbesondere in Form der Schräge, mittels einer Rotationsbewegung gegen das Öffnungsmittel, insbesondere in Form der Schräge, zu schieben und so zu öffnen. Um ein unbeabsichtigtes Öffnen des Behälters zu verhindern, kann die Vorrichtung des Verfahrens mit einer Transportsicherung ausgestaltet sein, welches ein Einschieben der Aufnahme oder ein Abknicken der Aufnahme verhindert, bis die Transportsicherung vor dem Verwenden der Vorrichtung des Verfahrens durch einen Anwender der Vorrichtung des Verfahrens entfernt wird.

Um ein Ausfließen der Flüssigkeitskomponente aus dem Behälter, insbesondere eines Behälters in Form einer Glasampulle, nach dessen Öffnung zu erleichtern, ist die Aufnahme vorzugsweise so ausgestaltet, dass der Behälter bei einem ordnungsgemäßen Aufstellen der Vorrichtung des Verfahrens auf einer waagrechten Oberfläche, beispielsweise einem Tisch, einen Winkel zu einer Senkrechten der Oberfläche in einem Bereich von 10° bis 30°, bevorzugt von 15° bis 25°, einnimmt.

Die Aufnahme der Vorrichtung des Verfahrens ist fluidleitend mit einem Reservoir verbunden. Dabei können die Aufnahme und das Reservoir unmittelbar fluidleitend zueinander ausgestaltet sein, oder zwischen der Aufnahme und dem Reservoir ist ein fluidleitendes Element, beispielsweise ein Rohr oder ein Schlauch angeordnet, welches eine fluidleitende Verbindung herstellt. Das Reservoir dient der kontaminationsfreien, im Wesentlichen sterilen, Zwischenlagerung der Flüssigkeitskomponente, welche nach dem Öffnen des Behälters aus diesem herausfließt, bis zu einem Anmischen mit einer Pulverkomponente als zweiter Ausgangskomponente des Knochenzementteigs. Das Reservoir lagert die Flüssigkeitskomponente kontaminationsfrei, und bei der Verwendung von Methymethacrylat als Flüssigkeitskomponente auch im Wesentlichen ohne Geruchsbelästigung für den Anwender der Vorrichtung des Verfahrens, derart, dass die Flüssigkeitskomponente einfach, schnell und im Volumen bedarfsgereicht entweder im Ganzen oder in mehreren, portionsweisen Entnahmevorgängen zur Anmischung des Knochenzementteigs entnehmbar ist. Dies erlaubt ein zeitversetztes Anmischen mehrerer Portionen an Knochenzementteig. Dadurch ist es möglich, immer nur eine derzeit notwendige Menge an Knochenzementteig bereitzustellen, diese zu verwenden und erst danach eine weitere Portion Knochenzementteig aus dem gleichen Behälter der Flüssigkeitskomponent anzumischen. Somit steht dem Operateur für jede dieser Portionen des Knochenzementteigs die gesamte Verarbeitungszeit des verwendeten Knochenzementteigs zur Verfügung, was bei einer initialen Anmischung des gesamten Behälters nicht möglich wäre.

Dabei ist das Reservoir vorzugsweise so dimensioniert, dass die Flüssigkeitskomponente vollständig innerhalb des Reservoirs gelagert werden kann. Beispielsweise fasst das Reservoir ein Volumen von bis zu 50 ml einer Flüssigkeit.

Zur Aufnahme der Flüssigkeitskomponente kann das Reservoir auf unterschiedliche Weise ausgestaltet sein. In einer Ausgestaltungsform ist das Reservoir schalenartig ausgestaltet, um die Komplexität der Vorrichtung des Verfahrens weitgehend zu reduzieren. Bevorzugt umfasst das Reservoir dabei eine der Aufnahme zugewandte fluidleitend geöffnete Reservoiröffnung Dies erlaubt ein einfaches Einfließen der Flüssigkeitskomponente aus dem Behälter in das schalenartige Reservoir über die der Aufnahme zugewandte Reservoiröffnung.

Vorzugsweise liegt das Reservoir bei ordnungsgemäßer Aufstellung der Vorrichtung des Verfahrens auf einer waagrechten Oberfläche räumlich tiefer, also näher an der waagrechten Oberfläche, als die Aufnahme, so dass die Flüssigkeitskomponente ohne Zutun eines Anwenders gemäß der Schwerkraft aus dem Behälter in das Reservoir fließbar ist.

Um zu verhindern, dass der Behälter oder Behälterfragmente nach dem Öffnen des Behälters, insbesondere Glasfragmente nach dem Öffnen eines Behälters in Form einer Glasampulle, in das Reservoir gelangen, kann zwischen der Aufnahme und dem Reservoir ein Rückhalteelement angeordnet sein. Das Rückhalteelement kann beispielsweise ein Sieb, eine offenporöse Platte, insbesondere eine offenporöse Kunststoffplatte, oder einen offenporösen Stift, insbesondere einen offenporösen Kunststoffstift, umfassen oder aus dem Vorgenannten bestehen.

Um die Flüssigkeitskomponente aus dem Reservoir in eine Spritze zur weiteren Verwendung zu fördern, umfasst die Vorrichtung des Verfahrens mindestens einen Anschluss, welcher fluidleitend über ein Leitungsmittel mit dem Reservoir verbunden ist.

Das Leitungsmittel kann unterschiedlich ausgeformt sein, um den Anschluss und das Reservoir fluidleitend zu verbinden. Beispielsweise kann das Leitungsmittel als Kanal ausgeformt sein. Vorzugsweise ist das Leitungsmittel derart mit dem Reservoir verbunden, dass bei einem ordnungsgemäßen Aufstellen der Vorrichtung des Verfahrens im Wesentlichen die gesamte im Reservoir vorhandene Flüssigkeitskomponente durch das Leitungsmittel in Richtung des Anschlusses gefördert werden kann. Beispielsweise ist das Leitungsmittel im Bereich des bei einem ordnungsgemäßen Aufstellen der Vorrichtung des Verfahrens räumlich tieftesten Punktes des Reservoirs angeordnet.

Über den Anschluss kann eine Spritze reversible fluidleitend mit dem Reservoir verbunden werden, um die im Reservoir zwischengelagerte Flüssigkeitskomponente in die Spritze zu fördern. Um die fluidleitende Verbindung von Vorrichtung des Verfahrens und Spritze herzustellen, kann der Anschluss auf unterschiedliche Weisen ausgestaltet sein. In einer Ausgestaltungsform ist der Anschluss als Gewinde ausgeformt, um mit einem entsprechenden Gegenstück an der Spritze die fluidleitende Verbindung herzustellen. Beispielsweise kann der Anschluss ein Innengewinde aufweisen, welches mit einem Außengewinde an einer Spritze die fluidleitende Verbindung ausformt. In einer weiteren Ausgestaltungsform formen der Anschluss und die Spritze eine Bajonettverbindung aus. In einer weiteren Ausgestaltungsform können der Anschluss und die Spritze durch teilweises Einschieben der Spritze in den Anschluss fluidleitend miteinander verbunden werden. Beispielsweise kann das Leitungsmittel als Schlauch ausgeformt sein, um das Reservoir und den Anschluss fluidleitend zu verbinden, und eine Spritze kann am Anschluss über ein abschnittsweises Einschieben der Spritze in den Schlauch die fluidleitende Verbindung von Anschluss und Spritze ausformen. Nach dem Fördern der Flüssigkeitskomponente kann die Spritze vom Anschluss zur weiteren Verwendung gelöst werden.

Um ein Eindringen von Behälterfragmenten, insbesondere von Glasfragmente entstanden durch Öffnen eines Behälters in Form einer Glasampulle, in eine mit dem Anschluss fluidleitend verbundene Spritze zu unterbinden, kann der Anschluss mit einer Filtereinheit ausgestattet sein. Die Filtereinheit kann beispielsweise ein Sieb, eine offenporöse Platte, insbesondere eine offenporöse Kunststoffplatte, oder einen offenporösen Stift, insbesondere einen offenporösen Kunststoffstift, umfassen oder aus dem Vorgenannten bestehen. In einer Ausgestaltungsform sind der Anschluss und die Filtereinheit irreversibel miteinander verbunden, beispielsweise über eine Klebeverbindung. In einer weiteren Ausgestaltungsform sind der Anschluss und die Filtereinheit reversibel miteinander verbunden, beispielsweise ist die Filtereinheit als ein mit dem Anschluss verbindbarer Adapter ausgeformt. Dabei kann der Adapter beispielsweise über eine Gewindeverbindung oder eine Bajonettverbindung mit dem Anschluss verbunden werden.

Der Adapter kann beispielsweise wiederum mit einer dem Anschluss abgewandten Seite fluidleitend mit der Spritze verbunden werden, so dass der Anschluss über den Adapter fluidleitend mit der Spritze verbunden ist.

Um ein versehentliches Fließen der Flüssigkeitskomponente über den mindestens einen Anschluss in eine mit dem mindestens einen Anschluss fluidleitend verbundene Spritze zu verhindern, ist es bevorzugt, dass, bei ordnungsgemäßer Aufstellung der Vorrichtung des Verfahrens auf einer waagrechten Oberfläche, das Reservoir räumlich tiefer, also näher an der waagrechten Oberfläche, liegt als der mindestens eine Anschluss.

Eine Ausführungsform der Vorrichtung des Verfahrens ist dadurch gekennzeichnet, dass die Vorrichtung des Verfahrens eine Vielzahl an Anschlüssen, insbesondere zwei, drei, vier oder fünf Anschlüsse, zum fluidleitenden Verbinden der Vorrichtung des Verfahrens mit einer Spritze, in welcher eine Pulverkomponente als zweite Ausgangskomponente des Knochenzementteigs lagerbar ist, und eine Vielzahl an Leitungsmitteln, insbesondere zwei, drei, vier oder fünf Leitungsmittel, umfasst, wobei jeweils eines der Leitungsmittel aus der Vielzahl der Leitungsmittel das Reservoir und einen der Anschlüsse aus der Vielzahl der Anschlüsse fluidleitend miteinander verbindet. Die Vorrichtung des Verfahrens umfasst eine Vielzahl an Leitungsmittel-Anschluss-Paarungen, um die Vorrichtung des Verfahrens fluidleitend mit einer Vielzahl an Spritzen gleichzeitig fluidleitend verbinden zu können. Durch die Vielzahl an Anschlüssen kann die Vorrichtung des Verfahrens bereits vor dem ersten Anmischen einer Portion Knochenzementteig mit einer Vielzahl an Spritzen fluidleitend verbunden werden, was einem Anwender der Vorrichtung des Verfahrens eine Verwendung mehrerer Spritzen im Zuge der Operation erleichtert. Zudem kann für den Fall einer Verstopfung eines der Anschlüsse auf einen anderen Anschluss zurückgegriffen werden.

Eine Ausführungsform der Vorrichtung des Verfahrens ist dadurch gekennzeichnet, dass das Reservoir in eine Vielzahl an Kompartimenten unterteilt ist, wobei jeweils eines der Kompartimente über eines der Leitungsmittel mit einem der Anschlüsse fluidleitend verbunden ist. Durch die Kompartimente ist das Reservoir in kleinere Unterreservoire unterteilt, was eine Vorportionierung der aus dem Behälter ausgeflossenen Flüssigkeitskomponente innerhalb des Reservoirs erlaubt. Da die einzelnen Kompartimente über jeweils ein separates Leitungsmittel mit einem separaten Anschluss fluidleitend verbunden sind, kann über jeden dieser separaten Anschlüsse eine vorportionierte Flüssigkeitskomponentenmenge aus der Vorrichtung des Verfahrens entnommen werden. So wird sichergestellt, dass immer nur eine gewünschte, vordefinierte Menge an Flüssigkeitskomponente aus einem der Anschlüsse entnehmbar ist, was dem Anwender der Vorrichtung des Verfahrens die Anwendung erleichtert.

In einer Ausführungsform weisen die einzelnen Kompartimente unterschiedliche Volumina auf, so dass aus den entsprechenden Anschlüssen der Vorrichtung des Verfahrens unterschiedliche Mengen der Flüssigkeitskomponente entnehmbar sind. Dies erlaubt die zeitlich gestaffelte Anmischung unterschiedlicher Portionen an Knochenzementteig aus einem Behälter enthaltend die Flüssigkeitskomponente. Die Portionen können sich beispielsweise Volumen und/oder Viskosität unterschieden.

Eine Ausführungsform der Vorrichtung des Verfahrens ist dadurch gekennzeichnet, dass die einzelnen Kompartimente jeweils ein im Wesentlichen gleichgroßes Volumen aufweisen. Dies erlaubt die zeitlich gestaffelte Anmischung im Wesentlichen gleichartiger Portionen an Knochenzementteig mittels eines einzigen Behälters der Flüssigkeitskomponente. Die Portionen können sich beispielsweise, bei gleichzeitiger Verwendung gleicher Teil an Pulverkomponente, in ihren Volumina und Viskositäten gleichen.

Um vorportionierte Mengen der Flüssigkeitskomponente über die einzelnen Anschlüsse zur Verfügung zu stellen, kann das Reservoir auf unterschiedliche Weisen in die Kompartimente unterteilt sein.

Eine Ausführungsform der Vorrichtung des Verfahrens ist dadurch gekennzeichnet, dass das Reservoir mittels mindestens einer Zwischenwand in die Kompartimente unterteilt ist. Beispielsweise kann eine einzelne Zwischenwand das Reservoir in zwei Kompartimente oder zwei sich kreuzende Zwischenwände das Reservoir in vier Kompartimente unterteilen. Zwischenwände erlauben eine einfache und kostengünstige Konstruktionsweise des in Kompartimente unterteilten Reservoirs.

Eine Ausführungsform der Vorrichtung des Verfahrens ist dadurch gekennzeichnet, dass die Kompartimente jeweils an einer der Aufnahme zugewandten oberen Kompartimentseite fluidleitend geöffnet sind, so dass die Kompartimente untereinander über die oberen Kompartimentseiten fluidleitend miteinander verbunden sind. Beispielsweise kann eine erste Zwischenwand das Reservoir, insbesondere ein schalenartiges Reservoir, welches eine der Aufnahme zugewandten fluidleitend geöffnete Reservoirseite aufweist, in zwei Kompartimente unterteilen, welche jeweils eine der Aufnahme zugewandte fluidleitend geöffnete obere Kompartimentseite aufweisen. Weiterhin kann das Reservoir beispielsweise durch eine zweite, die erste Zwischenwand kreuzende, Zwischenwand in vier Kompartimente mit entsprechend vier fluidleitend geöffneten oberen Kompartimentseiten unterteilt werden.

Das Leitungsmittel kann unterschiedlich ausgeformt sein, um das Reservoir oder die einzelnen Kompartimente des Reservoirs mit dem Anschluss oder den Anschlüssen fluidleitend zu verbinden.

Eine Ausführungsform der Vorrichtung des Verfahrens ist dadurch gekennzeichnet, dass das mindestens eine Leitungsmittel ein Schlauch ist. Ein Schlauch erlaubt eine einfache, günstige und flexible fluidleitende Verbindung von Reservoir und Anschluss.

Eine Ausführungsform der Vorrichtung des Verfahrens ist dadurch gekennzeichnet, dass der Schlauch einen Innendurchmesser in einem Bereich von 0,5 mm bis 3 mm, bevorzugt zwischen 0,5 mm und 2,5 mm, weiter bevorzugt zwischen 0,5 mm und 2 mm aufweist. Aufgrund der Oberflächenspannung der Flüssigkeitskomponente verhindert ein derartiger Innendurchmesser des Schlauchs ein selbstständiges Abfließen der Flüssigkeitskomponente aus dem Reservoir durch das Leitungsmittel in Richtung des Anschlusses. Die Flüssigkeitskomponente verbleibt mit einem derartigen Innendurchmesser des Schlauchs innerhalb des Reservoirs, bis der Anwender aktiv ein Fördern aus dem Reservoir in Richtung des Anschlusses vorantreibt, beispielsweise durch Ausübung eines Förderdrucks auf die Flüssigkeitskomponente oder durch Anlegen eines Unterdrucks durch das Leitungsmittel aus Richtung des Anschlusses, beispielsweise ausgeübt durch eine Betätigung einer mit dem Anschluss fluidleitend verbundener Spritze.

Das System des Verfahrens zum Bereitstellen eines Knochenzementteigs aus zwei Ausgangskomponenten umfasst eine Vorrichtung des Verfahrens, wobei in der Aufnahme ein Behälter, bevorzugt ein Behälter in der Form einer Glasampulle, enthaltend eine Flüssigkeitskomponente als erste Ausgangskomponente lagert und wobei das System des Verfahrens eine Vielzahl an Spritze, insbesondere zwei, drei, vier oder fünf Spritzen, umfasst, wobei die Spritzen jeweils eine Pulverkomponente als zweite Ausgangskomponente enthalten.

Unter einem Behälter werden alle Gefäße verstanden, welche die Flüssigkeitskomponente hermetisch dicht und steril lagern können und durch manuelle Krafteinwirkung zerstörbar sind. Beispiele für Behälter sind Glasampullen, Kunststoffampullen und Kunststoffbeutel. Aufgrund der leichten Sterilisierbarkeit und guten Öffenbarkeit durch manuelle Krafteinwirkung sind Glasampullen bevorzugt.

Das System des Verfahrens umfasst eine Vielzahl an Spritzen, welche eine Pulverkomponente als zweite Ausgangskomponente des Knochenzementteigs enthalten. Dies erlaubt ein einfaches und schnelles Anmischen des Knochenzementteigs nach dem Fördern der Flüssigkeitskomponente aus dem Behälter über das Reservoir, das Leitungsmittel und den Anschluss in eine der Spritzen direkt in dieser Spritze. Ein Umfüllen des Knochenzementteigs in eine der Spritzen entfällt dadurch. Zum Anmischen des Knochenzementteigs in der Spritze kann diese eine Mischeinrichtung, wie beispielsweise einen Mischstab oder eine oder mehrere Mischkugeln enthalten. Eine Mischkugel dient einem Anmischen des Knochenzementteigs durch Schütteln der Spritze, indem die Mischkugel mitsamt den beiden Ausgangskomponenten durch das Schütteln in der Spritze hin- und herbewegt und dadurch das Anmischen unterstützt wird.

In einer Ausführungsform des Systems des Verfahrens sind die Spritzen mischeinrichtungsfrei ausgestaltet.

Unter einer Spritze ist ein Behältnis zu verstehen, welches die Pulverkomponente kontaminationsfrei lagern kann und mittels dem ein Knochenzementteig an eine gewünschte Stelle appliziert werden kann. Bevorzugt umfasst die Spritze einen Spritzenkolben, welcher durch Vortreiben in Richtung einer Austragsöffnung der Spritze den Knochenzementteig aus der Spritze austragen kann. Zum Austragen des Knochenzementteigs kann an der Spritze, insbesondere an der Austragsöffnung, eine Austragshilfe, wie beispielsweise eine Kanüle, ein Austragsschnorchel oder ein Schlauch, befestigt werden.

Die Spritze ist reversibel fluidleitend mit dem Anschluss der Vorrichtung des Verfahrens verbindbar. Dazu kann die Spritze beispielsweise ein Gewinde, insbesondere ein Außengewinde, oder einen Teil einer Bajonettverbindung umfassen.

Um zu verhindern, dass mit der Flüssigkeitskomponente Fragmente des Behälters in die Spritze transferiert werden, kann die Spritze einen Filter umfassen. Der Filter kann beispielsweise ein Sieb, eine offenporöse Platte, insbesondere eine offenporöse Kunststoffplatte, oder einen offenporösen Stift, insbesondere einen offenporösen Kunststoffstift, umfassen oder aus dem Vorgenannten bestehen. In einer Ausgestaltungsform sind die Spritze und der Filter reversibel miteinander verbunden, beispielsweise ist Filter als ein mit der Spritze verbindbarer Spritzenadapter ausgeformt. Dabei kann der Spritzenadapter beispielsweise über eine Gewindeverbindung oder eine Bajonettverbindung mit der Spritze verbunden werden. Vor dem Applizieren des Knochenzementteigs aus der Spritze heraus wird der Filter vorzugweise von der Spritze, beispielsweise durch Abschrauben, entfernt. In einer weiteren Ausgestaltungsform ist der Filter im Bereich der Austragsöffnung in die Spritze eingeschoben und wird beim Austragen des Knochenzementteigs durch diesen aus der Spritze, insbesondere aus der Austragsöffnung, ausgeschoben.

Das System des Verfahrens ist vorzugsweise so ausgelegt, dass die enthaltene Flüssigkeitskomponente für ein Anmischen eines applizierbaren und für Operationen verwendbaren Knochenzementteigs in mehreren, vorzugsweise in allen, Spritzen des Systems des Verfahrens ausreicht. Beispielsweise enthält der Behälter 30 ml Flüssigkeitskomponente und die Vielzahl an Spritzen enthält insgesamt 45 g Pulverkomponente.

Das System des Verfahrens erlaubt ein zeitversetztes Anmischen mehrerer Portionen Knochenzementteig aus einem einzigen Gebinde, insbesondere mittels eines einzigen Behälters der Flüssigkeitskomponente. Dadurch wird der Zeitdruck auf einen Operateur reduziert, welcher ansonsten den gesamten initial angemischten Knochenzementteig innerhalb der Verarbeitungszeit desselben verwenden müsste. Die zeitversetzte, portionsweise Anmischung startet für jede der Spritzen eine separate Verarbeitungszeit, so dass insgesamt eine längere Zeitspanne für die Verwendung des Knochenzementteigs zur Verfügung steht.

Um ein versehentliches Fließen der Flüssigkeitskomponente über den mindestens einen Anschluss in eine mit dem mindestens einen Anschluss fluidleitend verbundene Spritze zu verhindern, ist es bevorzugt, dass, bei ordnungsgemäßer Aufstellung der Vorrichtung des Verfahrens auf einer waagrechten Oberfläche, das Reservoir räumlich tiefer, also näher an der waagrechten Oberfläche, liegt als der mindestens eine Anschluss. Zudem ist es bevorzugt, dass die Flüssigkeitskomponente nach dem Fließen in das Reservoir bei ordnungsgemäßer Aufstellung der Vorrichtung des Verfahrens auf einer waagrechten Oberfläche einen Flüssigkeitsspiegel aufweist, welcher räumlich tiefer liegt als der mindestens eine Anschluss.

Eine Ausführungsform des Systems des Verfahrens ist dadurch gekennzeichnet, dass die Vorrichtung des Verfahrens eine Vielzahl an Anschlüssen, insbesondere zwei, drei, vier oder fünf Anschlüsse, aufweist. Dabei ist es bevorzugt, dass das System des Verfahrens genauso viele Spritzen wie Anschlüsse aufweist. Beispielsweise weist das System des Verfahrens zwei Anschlüsse und zwei Spritzen auf.

Dies erlaubt ein gleichzeitig fluidleitendes Verbinden aller Spritzen mit der Vorrichtung des Verfahrens bereits vor dem Beginn einer Operation, was den Operationsablauf für den Operateur erleichtert.

Eine Ausführungsform des Systems des Verfahrens ist dadurch gekennzeichnet, dass die Spritzen reversibel fluidleitend mit den Anschlüssen verbunden sind. Dies verschließt sowohl die Vorrichtung des Verfahrens als auch die Spritzen kontaminationsfrei, so dass das System des Verfahrens, insbesondere der Behälter und die Pulverkomponente innerhalb des Systems des Verfahrens, steril lagerbar ist. Insbesondere kann das derartige System des Verfahrens aus einer sterilen Packung entnommen werden, ohne dass dies ein Kontaminationsrisiko für das System des Verfahrens, insbesondere des Behälters und der Pulverkomponente innerhalb des Systems des Verfahrens, darstellt. Zudem verringern sich dadurch mögliche Anwendungsfehler des Systems des Verfahrens durch einen Operateur.

Eine Ausführungsform des Systems des Verfahrens, wobei die Vorrichtung des Systems des Verfahrens eine Vorrichtung nach einer der vorhergehenden Ausführungsformen der Vorrichtung des Verfahrens ist, welche ein Reservoir aufweisen, welches mittels mindestens einer Zwischenwand in eine Vielzahl an Kompartimenten unterteilt ist und wobei die Kompartimente der Vorrichtung des Verfahrens des Systems des Verfahrens jeweils an einer der Aufnahme zugewandten oberen Kompartimentseite fluidleitend geöffnet sind, so dass die Kompartimente über die oberen Kompartimentseiten fluidleitend miteinander verbunden sind und wobei jeweils eines der Kompartimente über eines der Leitungsmittel mit einem der Anschlüsse der Vorrichtung des Verfahrens fluidleitend verbunden ist, ist dadurch gekennzeichnet, dass im Behälter eine derartige Menge an Flüssigkeitskomponente lagert, dass nach einem Öffnen des Behälters alle Kompartimente des Reservoirs mit der Flüssigkeitskomponente befüllbar sind und dabei jeweils eine Füllhöhe aufweisen, welche eine Höhe der die jeweiligen Kompartimente trennenden mindestens einen Zwischenwand zumindest abschnittsweise überragt.

Dies erlaubt ein Befüllen aller Kompartimente mit der Flüssigkeitskomponente, da sich die aus dem Behälter in das Reservoir einfließende Flüssigkeitskomponente über die oberen Kompartimentseiten über die jeweils trennende Zwischenwand hinweg verteilt.

Beispielsweise fließt die Flüssigkeitskomponente aus dem Behälter lediglich in eines der Kompartimente des Reservoirs und füllt dieses. Ist das Volumen dieses Kompartiments gefüllt, wird weitere, in dieses Kompartiment fließende Flüssigkeitskomponente aus dem bereits gefüllten Kompartiment über die Zwischenwand des Kompartiments in ein angrenzendes Kompartiment überfließen. Auf diese Weise kann sich die Monomerflüssigkeit nacheinander in alle Kompartimente des Reservoirs verteilen und diese befüllen. Die Menge an Flüssigkeitskomponente ist dabei so abgestimmt, dass ein vollständiges Befüllen aller Kompartimente möglich ist und die Flüssigkeitskomponente jeweils einen Füllstand in den Kompartimenten aufweist, welcher zumindest abschnittsweise höher ist als die entsprechende Zwischenwand. Dies erlaubt ein "Angleichen" der Füllhöhe der Monomerflüssigkeit über alle Kompartimente hinweg. Die Kompartimente weisen somit einen Überstand auf, welcher die zumindest eine Zwischenwand überragt.

Eine Ausführungsform des Systems des Verfahrens ist dadurch gekennzeichnet, dass die Füllhöhe der Kompartimente zumindest abschnittsweise die Höhe der zumindest einen Zwischenwand um nicht mehr als 1 mm überragt. In anderen Worten ist der Überstand nicht höher als 1 mm. Dies erlaubt ein gutes Verteilen der Flüssigkeitskomponente in alle Kompartimente. Der Überstand fließt mit dem Entleeren des ersten Kompartiments in dieses hinein und wird mitentleert. Dies sorgt beispielsweise dafür, dass trotz gleich großer Kompartimente das zuerst geleerte Kompartiment den größten Anteil an Flüssigkeitskomponente liefert. Durch den geringen Überstand von maximal 1 mm wird sichergestellt, dass die Kompartimente unabhängig von der Reihenfolge ihrer Entleerung im Wesentlichen das durch die Kompartimente vordefinierte Volumen an Flüssigkeitskomponente bereitstellen.

Eine Ausführungsform des Systems des Verfahrens ist dadurch gekennzeichnet, dass in den Spritzen eine im Wesentlichen gleiche Menge der Pulverkomponente gelagert ist. Dies verhindert Verwechslungsgefahren der einzelnen Spritzen, welche unter Umständen Knochenzemente mit unterschiedlichen Eigenschaften, wie beispielsweise ihrer Rheologie, liefern würden. Bevorzugt weisen zudem die Kompartimente im Wesentlichen gleich große Volumina auf, so dass mit jeder Spritze ein Knochenzementteig mit im Wesentlichen gleichen Eigenschaften, insbesondere im Wesentlichen gleicher Rheologie, erhältlich ist.

Unter einem Knochenzementteig wird eine Substanz verstanden, die geeignet ist im Bereich der Medizintechnik eine stabile Verbindung zwischen künstlichen Gelenken, wie beispielsweise Hüft- und Kniegelenken, und Knochenmaterial zu erstellen und/oder Wirbelkörper zu stabilisieren. Durch Aushärtung wird aus einem Knochenzementteig ein Knochenzement. Bevorzugt handelt es sich bei diesen Knochenzementen um Polymethylmethacrylat-Knochenzemente (PMMA-Knochenzemente) oder um anorganische Knochenzemente. PMMA-Knochenzemente kommen schon lange in medizinischen Anwendungen zum Einsatz und gehen auf Arbeiten von Sir Charnley zurück (vgl. Charnley, J. Anchorage of the femoral head prosthesis of the shaft of the femur. J. Bone Joint Surg. 1960; 42, 28-30.). PMMA-Knochenzemente können dabei aus einer Pulverkomponente umfassend ein Knochenzementpulver als erster Ausgangskomponente und einer Flüssigkeitskomponente umfassend eine Monomerflüssigkeit als zweiter Ausgangskomponente hergestellt werden. Bei geeigneter Zusammensetzung können die beiden Ausgangskomponenten getrennt voneinander lagerstabil sein. Bei Inkontaktbringen der beiden Ausgangskomponenten entsteht durch Quellung der Polymerbestandteile des Knochenzementpulvers ein plastisch verformbarer Knochenzementteig. Dabei wird eine Polymerisation des Monomers durch Radikale eingeleitet. Mit fortschreitender Polymerisation des Monomers erhöht sich die Viskosität des Knochenzementteigs, bis dieser vollständig aushärtet.

Unter einem Knochenzementpulver wird ein Pulver verstanden, welches mindestens ein partikuläres Polymethylmethacrylat und/oder ein partikuläres Polymethylmethacrylat-Copolymer umfasst. Beispiele für Copolymere sind Styren und/oder Methylacrylat. In einer Ausgestaltungsform kann das Knochenzementpulver zusätzlich ein hydrophiles Additiv umfassen, welches die Verteilung der Monomerflüssigkeit innerhalb des Knochenzementpulvers unterstützt. In einer weiteren Ausgestaltungsform kann das Knochenzementpulver zusätzlich einen Initiator, welcher die Polymerisation einleitet, umfassen. In einer weiteren Ausgestaltungsform kann das Knochenzementpulvers zusätzlich einen Röntgenopaker umfassen. In noch einer weiteren Ausgestaltungsform kann das Knochenzementpulver zusätzlich pharmazeutisch aktive Substanzen, wie beispielsweise Antibiotika, umfassen.

Bevorzugt umfasst das Knochenzementpulver als hydrophiles Additiv mindestens ein partikuläres Polymethylmethacrylat und/oder ein partikuläres Polymethylmethacrylat-Copolymer, einen Initiator und einen Röntgenopaker oder besteht aus diesen Komponenten.

Weiter bevorzugt umfasst das Knochenzementpulver mindestens ein partikuläres Polymethylmethacrylat und/oder ein partikuläres Polymethylmethacrylat-Copolymer, einen Initiator, einen Röntgenopaker und ein hydrophiles Additiv oder besteht aus diesen Komponenten. Am bevorzugtesten umfasst das Knochenzementpulver mindestens ein partikuläres Polymethylmethacrylat und/oder ein partikuläres Polymethylmethacrylat-Copolymer, einen Initiator, einen Röntgenopaker, ein hydrophiles Additiv und ein Antibiotikum oder besteht aus diesen Komponenten.

Erfindungsgemäß kann die Partikelgröße des partikulären Polymethylmethacrylat und/oder des partikulären Polymethylmethacrylat-Copolymers des Knochenzementpulvers der Siebfraktion kleiner 150 µm, bevorzugt kleiner 100 µm, entsprechen.

Erfindungsgemäß kann das hydrophile Additiv partikulär und/oder faserförmig ausgestaltet sein. In einer weiteren Ausgestaltungsform kann das hydrophile Additiv schwerlöslich, bevorzugt unlöslich, in Methylmethacrylat sein. In einer weiteren Ausgestaltungsform kann das hydrophile Additiv ein Aufsaugvermögen von mindestens 0,6 g Methylmethacrylat pro Gramm hydrophiles Additiv besitzen. In einer weiteren Ausgestaltungsform kann das hydrophile Additiv eine chemische Substanz mit mindestens einer OH-Gruppe aufweisen. Dabei kann bevorzugt vorgesehen sein, dass das hydrophile Additiv kovalent gebundene OH-Gruppen an seiner Oberfläche besitzt. Beispiele für solche bevorzugten hydrophilen Additive können Additive ausgewählt aus der Gruppe umfassend Cellulose, Oxycellulose, Stärke, Titandioxid und Siliziumdioxid sein, wobei pyrogenes Siliziumdioxid besonders bevorzugt ist. In einer Ausgestaltungsform kann die Partikelgröße des hydrophilen Additivs der Siebfraktion kleiner 100 µm, bevorzugt kleiner 50 µm und am bevorzugtesten kleiner 10 µm entsprechen. Das hydrophile Additiv kann in einer Menge von 0,1 bis 2,5 Gew.-% bezogen auf das Gesamtgewicht des Knochenzementpulvers enthalten sein.

Erfindungsgemäß kann der Initiator Dibenzoylperoxid enthalten oder aus Dibenzoylperoxid bestehen.

Erfindungsgemäß versteht man unter einem Röntgenopaker eine Substanz, welche es erlaubt, den Knochenzement auf röntgendiagnostischen Aufnahmen sichtbar zu machen. Beispiele für Röntgenopaker können Bariumsulfat, Zirkondioxid und Kalziumcarbonat umfassen. Erfindungsgemäß kann die pharmazeutisch aktive Substanz ein oder mehrere Antibiotika und gegebenenfalls zugesetzte Co-Faktoren für das eine oder die mehreren Antibiotika umfassen. Bevorzugt besteht die pharmazeutisch aktive Substanz aus einem oder mehreren Antibiotika und gegebenenfalls zugesetzten Co-Faktoren für das eine oder die mehreren Antibiotika. Beispiele für Antibiotika sind unter anderem Gentamicin, Clindamycin und Vancomycin. Erfindungsgemäß kann die Monomerflüssigkeit das Monomer Methylmethacrylat umfassen oder aus Methylmethacrylat bestehen. In einer Ausgestaltungsform umfasst die Monomerflüssigkeit neben dem Monomer einen darin gelösten Aktivator, wie beispielsweise N,N-Dimethyl-p-toluidin, oder besteht aus Methylmethacrylat und N,N-Dimethyl-p-toluidin. Unter einem anorganischen Knochenzement wird ein Knochenzement auf Basis von Calciumphosphaten und Calciumsulfat-Dihydrat verstanden. Als Pulverkomponente kommen dabei Pulver von Calciumphosphaten und/oder Calciumsulfat-Dihydrat zum Einsatz, welche durch eine Flüssigkeitskomponente umfassend eine wässrige Lösung verschiedener Salze aushärtbar ist. Es wurde eine Vielzahl an anorganischen Knochenzementen beschrieben, von denen exemplarisch folgende genannt sind: EP 1 592 463 B1, EP 2 271 585 B1 und EP 2 988 789 B1.

### Figuren

Die Erfindung wird im Folgenden durch Figuren weiter beispielhaft illustriert. Die Erfindung ist nicht auf die Figuren beschränkt.

Es zeigen
- Fig. 1: einen schematischen Längsschnitt einer Vorrichtung zum Bereitstellen einer Flüssigkeitskomponente als einer ersten Ausgangskomponente eines Knochenzementteigs aus zwei Ausgangskomponenten enthaltend einen Behälter mit der Flüssigkeitskomponente,
- Fig. 2: einen schematischen Längsschnitt eines Systems zum Bereitstellen eines Knochenzementteigs umfassend die Vorrichtung aus Figur 1, den Behälter aus Figur 1 sowie eine erste Spritze und eine zweite Spritze,
- Fig. 3: das System aus Figur 2 mit geöffnetem Behälter,
- Fig. 4: das System aus den Figuren 2 und 3 beim Fördern eines Teils der Flüssigkeitskomponente in die erste Spritze,
- Fig. 5: das System aus den Figuren 2 bis 4 mit fluidleitend getrennter erster Spritze,
- Fig. 6: das System aus den Figuren 2 bis 5 mit fluidleitend getrennter zweiter Spritze,
- Fig. 7: einen schematischen Längsschnitt eines weiteren Systems zum Bereitstellen eines Knochenzementteigs aus zwei Ausgangskomponenten,
- Fig. 8: einen schematischen Längsschnitt eines weiteren Systems zum Bereitstellen eines Knochenzementteigs aus zwei Ausgangskomponenten umfassend ein in Kompartimente unterteiltes Reservoir und einen Behälter enthaltend eine Flüssigkeitskomponente,
- Fig. 9: eine schematische Aufsicht auf einen Ausschnitt des Systems aus Fig. 8 umfassend das Reservoir,
- Fig. 10: eine schematische Seitenansicht des Reservoirs aus den Fig. 8 und 9,
- Fig. 11: das System aus den Figuren 8 bis 10 mit geöffnetem Behälter,
- Fig. 12: das System aus den Figuren 8 bis 11 nach einem Fördern eines Teils der Flüssigkeitskomponente in eine erste Spritze, und
- Fig. 13: ein Flussdiagramm eines Verfahrens zum Bereitstellen eines Knochenzementteigs.

### Beschreibung der Figuren

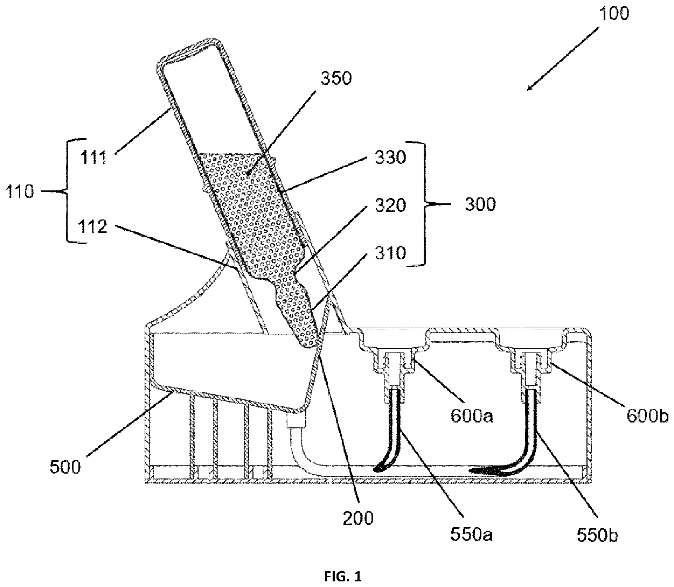

**Figur 1** zeigt einen schematischen Längsschnitt einer beispielhaften Ausführung einer Vorrichtung 100 des Verfahrens zum Bereitstellen einer Flüssigkeitskomponente als einer ersten Ausgangskomponente eines Knochenzementteigs aus zwei Ausgangskomponenten. Die Vorrichtung 100 umfasst eine rohrartige Aufnahme 110, in welcher ein Behälter 300 enthaltend eine Flüssigkeitskomponente 350 als erste Ausgangskomponente des Knochenzementteigs gelagert ist. Der Behälter 300 ist eine Glasampulle mit einem Glasampullenkopf 310, welcher über einen Glasampullenhals 320 mit einem Glasampullenkörper 330 verbunden ist. Die Aufnahme 110 umfasst den Behälter 300 manschettenartig, so dass dieser sicher in der Vorrichtung 100 transportierbar ist. Die Aufnahme 110 ist unmittelbar fluidleitend mit einem schalenartig ausgeformten Reservoir 500 verbunden. Um den Behälter 300 zu öffnen, weist die Aufnahme 110 einen hinteren Aufnahmeabschnitt 111 auf, welcher in einen vorderen Aufnahmeabschnitt 112 einschiebbar ist, so dass der Behälter 300, insbesondere der Glasampullenkörper 330 gegen ein Öffnungsmittel 200 in Form einer Schräge schiebbar gelagert ist. Das Öffnungsmittel 200 ist in der gezeigten Ausführungsform als Teil einer Wand des Reservoirs 500 ausgestaltet.

Die Vorrichtung 100 umfasst einen ersten Anschluss 600a und einen zweiten Anschluss 600b, über welche die Vorrichtung 100 mit Spritzen, insbesondere mit zwei Spritzen gleichzeitig, insbesondere über zusammenschrauben reversibel fluidleitend verbindbar ist. die Der erste Anschluss 600a ist über ein erstes Leitungsmittel 550a und der zweite Anschluss 600b ist über ein zweites Leitungsmittel 550b fluidleitend mit dem Reservoir 500 verbunden. Die beiden Leitungsmittel 550a, 550b sind in Form zweier separater Schläuche ausgestaltet, welche beide einen separaten Zugang zum Reservoir ausbilden.

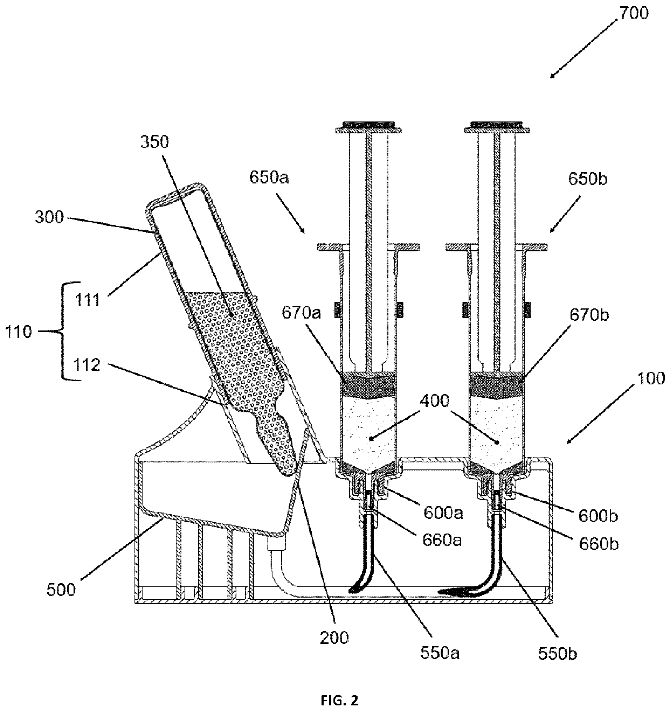

**Figur 2** zeigt ein System 700 zur Bereitstellung eines Knochenzementteigs aus zwei Ausgangskomponenten umfassend die Vorrichtung 100 aus Figur 1, den mit der Flüssigkeitskomponente 350 als erste Ausgangskomponente des Knochenzementteigs gefüllten Behälter 300 aus Figur 1 sowie eine erste Spritze 650a und eine zweite Spritze 650b.

Die Spritzen 650a, 650b enthalten eine Pulverkomponente 400 als zweite Ausgangskomponente des Knochenzementteigs und sind jeweils mit einem Spritzenkolben 670a, 670b ausgerüstet, welcher reversibel entlang einer Längsachse der Spritzen 650a, 650b verschiebbar gelagert ist. Die Spritzen 650a, 650b sind weiterhin mit jeweils einem fluidleitenden Filter 660a, 660b ausgestattet, so dass zwar Fluide, insbesondere Gase und die Flüssigkeitskomponente 350, aber keine Feststoffe, insbesondere die Pulverkomponente 400 und/oder Teile des Behälters 300, zwischen den Spritzen 650a, 650b und der Vorrichtung 100 über die Leitungsmittel 550a, 550b austauschbar sind. Die erste Spritze 650a ist über den ersten Anschluss 600a und die zweite Spritze 650b ist über den zweiten Anschluss 600b reversible fluidleitend mit der Vorrichtung 100 verbunden, wobei die Spritzen 650a, 650b mit den Anschlüssen 600a, 600b über Gewinde verbunden sind.

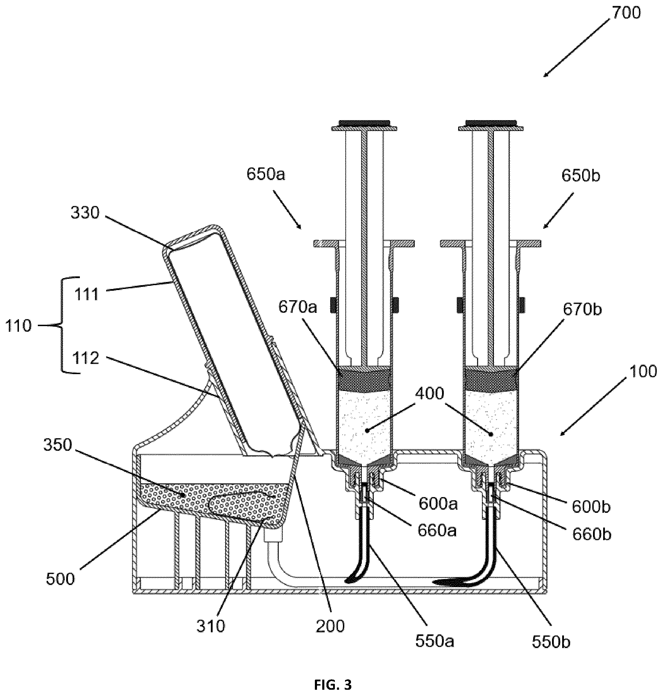

**Figur 3** zeigt das System 700 aus Figur 2, wobei im Vergleich zu Figur 2 der hintere Aufnahmeabschnitt 111 in den vorderen Aufnahmeabschnitt 112 abschnittsweise eingeschoben ist. Durch das Einschieben des hinteren Aufnahmeabschnitts 111 in den vorderen Aufnahmeabschnitt 112 ist der Behälterkopf 310 gegen das Öffnungsmittel 200 geschoben worden und dadurch abgebrochen, wodurch die Flüssigkeitskomponente 350 in das Reservoir 500 geflossen ist. Um ein Ausfließen der Flüssigkeitskomponente 350 aus dem Behälter 300 zu erleichtern, ist dieser in der Aufnahme 110 in einem Winkel von ungefähr 20° zu einer Senkrechten der Vorrichtung 100 angeordnet. Aufgrund der Oberflächenspannung der Flüssigkeitskomponente 350 verbleibt die Flüssigkeitskomponente 350 im Reservoir 500 und fließt nicht selbständig in Richtung der Spritzen 650a, 650b durch die Leitungsmittel 550a, 550b. Die Flüssigkeitskomponente 350 ist im Reservoir 500 somit kontaminationsfrei zwischengelagert und kann bedarfsgerecht und zeitlich unabhängig und versetzt in die Spritzen 650a, 650b gefördert werden.

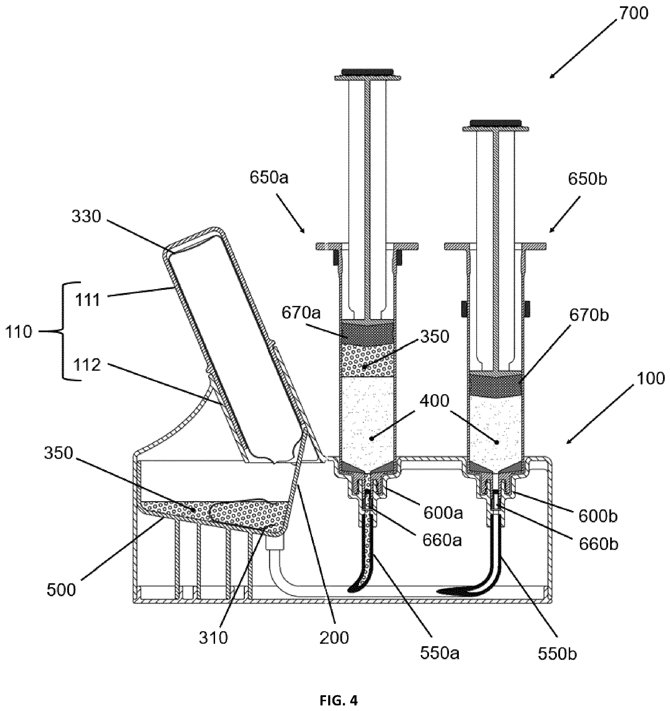

**Figur 4** zeigt das System 700 aus den Figuren 2 und 3, wobei im Vergleich zu Figur 2 ein Teil der Flüssigkeitskomponente 350 aus dem Reservoir 500 über das erste Leitungsmittel 550a in die erste Spritze 650a gefördert wurde. Zum Fördern der Flüssigkeitskomponente 350 wurde der Spritzenkolbens 670a der ersten Spritzen 650a teilweise aus einem dem ersten Anschluss 600a axial entgegengesetztem Ende der ersten Spritze 650a herausgezogen, wodurch ein Unterdruck in der ersten Spritze 650a das Fördern des Teils der Flüssigkeitskomponente 350 aus dem Reservoir 500 auslöste. Je weiter der Spritzenkolben 670a der ersten Spritze 650a herausgezogen wird, desto mehr Flüssigkeitskomponente 350 wird in die erste Spritze 650a gefördert. Zum Bestimmen der geförderten Menge an Flüssigkeitskomponente 350 kann ein Anwender des Systems beispielsweise eine Skala an einer Außenseite der ersten Spritze 650a (nicht gezeigt) ablesen. In einer weiteren, nicht gezeigten Ausführungsform weist das System 700 an den Spritzen 650a, 650b einen Kolbenhubregulierer auf, so dass die Spritzenkolben 670a, 670b lediglich bis zu einer vordefinierten Höhe aus den Spritzen 650a, 650b zum Fördern der Flüssigkeitskomponente 350 aus dem Reservoir 500 herausgezogen werden können. Der Kolbenhubregulierer erlaubt das Fördern einer vorbestimmten Menge der Flüssigkeitskomponente 350 in die Spritzen 650a, 650b, ohne dass der Anwender des Systems 700 eine Skala ablesen muss. Der Kolbenhubregulierer kann an die Menge an Pulverkomponente 400 in den Spritzen 650a, 650b angepasst werden, so dass ein Knochenzementteig mit einer gewünschten Rheologie in den Spritzen 650a, 650b bereitgestellt werden kann.

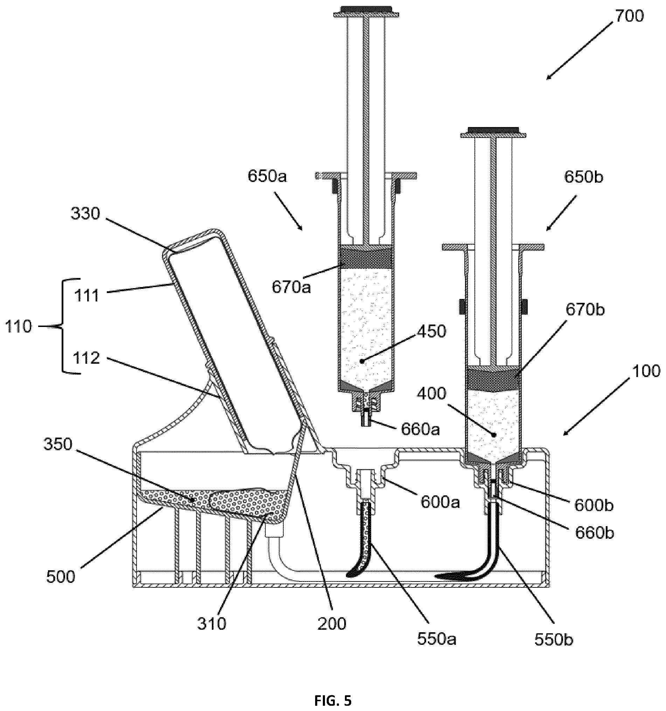

**Figur 5** zeigt das System 700 aus den Figuren 2 bis 4, wobei im Vergleich zu Figur 4 die erste Spritze 650a fluidleitend von der Vorrichtung 100 getrennt wurde. In der ersten Spritze 650a hat sich durch Schütteln der ersten Spritze 650a aus den zwei Ausgangskomponenten in der ersten Spritze 650a ein Knochenzementteig 450 ausgebildet, welcher innerhalb seines Verarbeitungszeitraums verwendet werden kann. Innerhalb des ersten Leitungsmittels 550a befinden sich noch Reste der Flüssigkeitskomponente 350, welche nicht in die erste Spritze 650a gefördert wurden. Im Reservoir 500 befindet sich ein verbliebener Teil der Flüssigkeitskomponente 350, welcher jederzeit für ein Fördern durch das zweite Leitungsmittel 550b in die zweite Spritze 650b zur Verfügung steht. Das Fördern der Flüssigkeitskomponente 350 in die zweite Spritze 670b kann auf die gleiche Weise wie das Fördern in die erste Spritze 650a vonstattengehen.

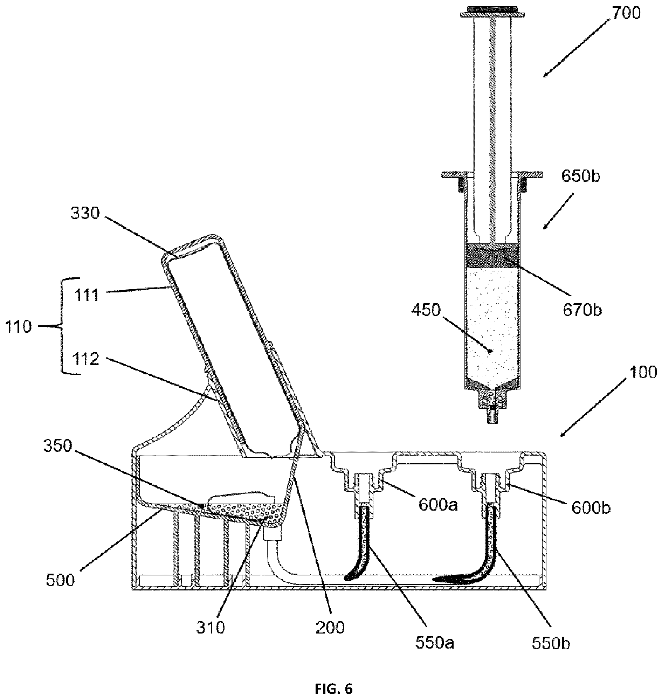

**Figur 6** zeigt das System 700 aus den Figuren 2 bis 5, wobei die erste Spritze 650a nicht mehr gezeigt ist. Im Vergleich zu Figur 5, wurde in Figur 6 ein weiterer Teil der Flüssigkeitskomponente 350 aus dem Reservoir 500 über das zweite Leitungsmittel 550b und den zweiten Anschluss 600b in die zweite Spritze 650b gefördert, die zweite Spritze 650b daraufhin vom zweiten Anschluss 600b fluidleitend getrennt und mittels Schüttelns der zweiten Spritze 650b die zwei Ausgangskomponenten in der zweiten Spritze 650b unter Ausbildung des Knochenzementteigs 450 gemischt. Der in der zweiten Spritze 650b bereitgestellte Knochenzementteig 450 kann in einem Verarbeitungszeitraum verwendet werden, welcher unabhängig vom Verarbeitungszeitraum des in der ersten Spritze 650a bereitgestellten Knochenzementteigs 450 (vgl. Figur 5) ist. Der Verarbeitungszeitraum des Knochenzementteigs 450 in den beiden Spritzen 650a, 650b des Systems 700 ist nicht zur gleichen Zeit gestartet. Das System 700 ermöglicht somit ein Bereitstellen zweiter Portionen des Knochenzementteigs 450, welche zeitlich unabhängig voneinander verwendbar sind. In weiteren, nicht gezeigten Ausführungsformen kann das System 700 mehr als zwei Anschlüsse 600a, 600b und mehr als zwei Spritzen 650a, 650b aufweisen, um mehr als zwei Portionen Knochenzementteig 450 bereitstellen zu können.

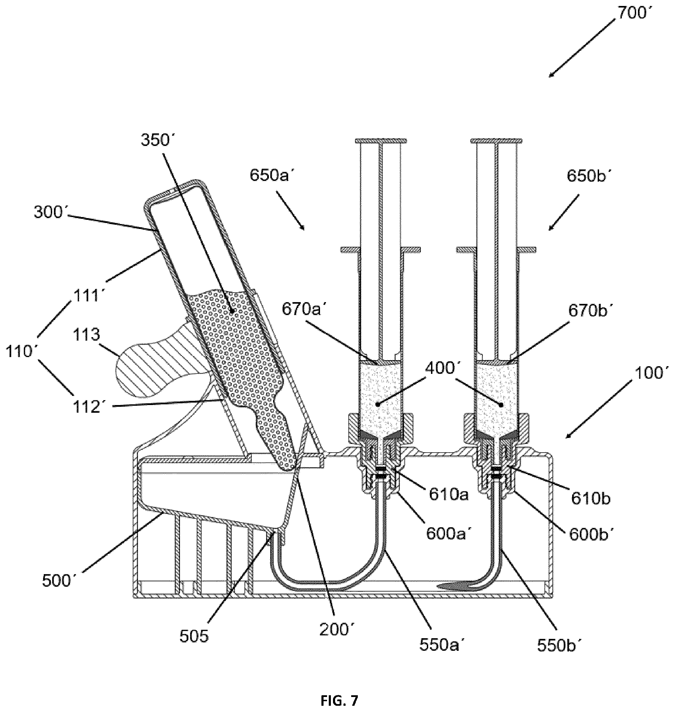

**Figur 7** zeigt einen schematischen Längsschnitt einer weiteren beispielhaften Ausführungsform eines Systems 700' zum Bereitstellen eines Knochenzementteigs aus zwei Ausgangskomponenten umfassend eine Vorrichtung 100', einen Behälter 300' enthaltend eine Flüssigkeitskomponente 350'als erste Ausgangskomponente sowie eine erste Spritze 650a'und eine zweite Spritze 650b' enthaltend eine Pulverkomponente 400' als zweite Ausgangskomponente. Die Ausführungsform des Systems 700' stimmt weitgehend mit der vorstehend beschriebenen und in den Figuren 2 bis 6 dargestellten Ausführungsform überein, so dass zur Vermeidung von Wiederholungen auf die vorstehende Beschreibung verwiesen wird. Abwandlungen einer gegenüber der in den Figuren 2 bis 6 gezeigten Ausführungsform weisen dasselbe Bezugszeichen mit einem zusätzlichen Apostroph auf.

Die Vorrichtung 100' weist an der Aufnahme 110', insbesondere am hinteren Aufnahmeabschnitt 111', eine Transportsicherung auf, welche ein Einschieben des hinteren Aufnahmeabschnitts 111' in den vorderen Aufnahmeabschnitt 112' verhindert. So wird sichergestellt, dass der Behälter 300' nicht unbeabsichtigt, beispielsweise beim Transport des Systems 770', geöffnet wird. Die Transportsicherung 113 umschließt die Aufnahme 110' manschettenartig und kann durch einfaches Ziehen von dieser entfernt werden, um ein Einschieben des hinteren Aufnahmeabschnitts 111' in den vorderen Aufnahmeabschnitt 112' zu ermöglichen.

Die beiden Spritzen 650a', 650b' des Systems 700' weisen im Gegensatz zu den beiden Spritzen 650a, 650b des Systems 700 aus den Figuren 2 bis 6 keine Filter 660a, 660b auf. Stattdessen ist die Vorrichtung 100' mit jeweils einer fluidleitenden Filtereinheit 610a, 610b in Form eines auf die beiden Anschlüsse 600a', 600b' anbringbaren Adapters ausgestattet. Die Filtereinheiten 610a, 610b sind zwischen den Spritzen 650a', 650b' und den Anschlüssen 600a', 600b' angeordnet, so dass Gase und die Flüssigkeitskomponente 350' vom Reservoir 500' in die Spritzen 650a', 650b' gefördert werden kann, Feststoffe, wie beispielsweise die Pulverkomponente 400' oder Teile des Behälters 300', aber nicht passieren können. Die Verwendung des Systems 700' zum Bereitstellen des Knochenzementteigs, insbesondere zweiter Portionen des Knochenzementteigs, stimmt weitgehend mit der Verwendung des Systems 700 aus den Figuren 2 bis 6 überein.

Figur 7 zeigt, im Gegensatz zu den vorhergehenden Figuren, eine das Reservoir 500 und das erste Leitungsmittel 550a' fluidleitend verbindende Reservoirdurchführung 505. Solch eine Reservoirdurchführung 505 verbindet auch das zweite Leitungsmittel 550b' des Systems 700' sowie die beiden Leitungsmittel 550a, 550b der Vorrichtung 100 aus den Figuren 1 bis 6 (jeweils nicht gezeigt).

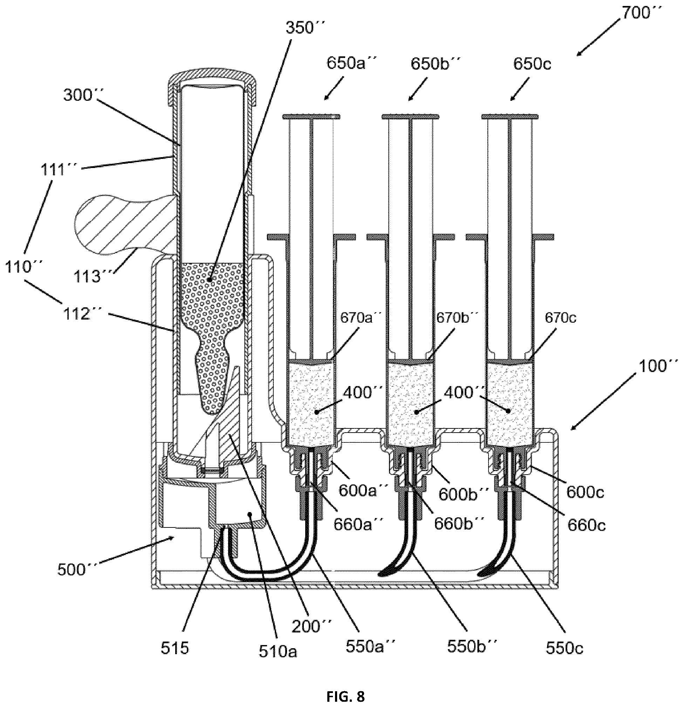

**Figur 8** zeigt einen schematischen Längsschnitt einer weiteren beispielhaften Ausführungsform eines Systems 700" zum Bereitstellen eines Knochenzementteigs aus zwei Ausgangskomponenten. Die Ausführungsform des Systems 700" stimmt weitgehend mit den vorstehend beschriebenen und in den Figuren 2 bis 6 sowie in Figur 7 dargestellten Ausführungsformen überein, so dass zur Vermeidung von Wiederholungen auf die vorstehende Beschreibung verwiesen wird. Abwandlungen einer gegenüber der in den Figuren 2 bis 6 beziehungsweise Figur 7 gezeigten Ausführungsform weisen dasselbe Bezugszeichen mit zwei Apostrophen auf.

Das System 700" weist im Vergleich zu den vorstehend beschriebenen Ausführungsformen neben dem ersten Anschluss 660a" und dem zweiten Anschluss 600b" einen dritten Anschluss 600c auf, welcher über ein drittes Leitungsmittel 550c fluidleitend mit dem Reservoir 500" verbunden ist. Das dritte Leitungsmittel 550c ist separat von den anderen beiden Leitungsmitteln 550a", 550b" ausgestaltet. Der dritte Anschluss 600c ist reversibel fluidleitend mit einer dritten Spritze 650c verbunden, wobei die dritte Spritze 650c, wie die beiden anderen Spritzen 650a", 650b", die Pulverkomponente 400" enthält und mit einem fluidleitenden Filter 660c ausgestattet ist, um ein Passieren von Feststoffen, insbesondere der Pulverkomponente 400" und/oder Teilen des Behälters 300", zwischen dritter Spritze 650c und Vorrichtung 100" zu unterbinden. Die dritte Spritze 650c weist einen axial in der dritten Spritze 650c verschiebbaren Spritzenkolben 670c.

Zwischen dem Behälter 300" und dem Reservoir 500" ist in der Aufnahme 110", insbesondere in dem vorderen Aufnahmeabschnitt 112" das Öffnungsmittel 200" in Form einer Schräge angeordnet, wobei dieses im Gegensatz zu den vorhergehenden Ausführungsformen nicht als Teil des Reservoirs 500" ausgestaltet ist.

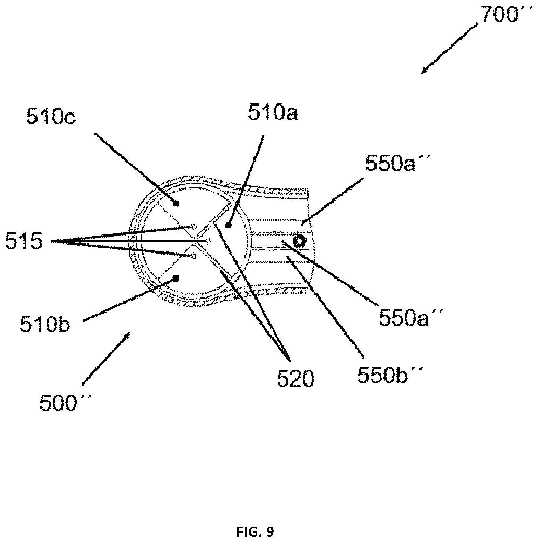

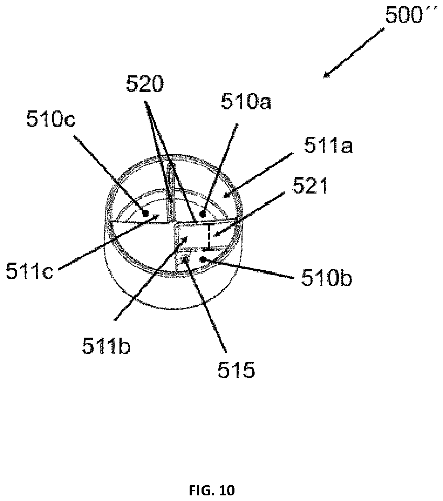

Das Reservoir 500" ist in drei Kompartimente 510a, 510b, 510c unterteilt, wobei in Figur 8 lediglich ein erstes Kompartiment 510a der drei Kompartimente 510a, 510b, 510c sichtbar ist. Jedes der Kompartimente 510a, 510b, 510c ist fluidleitend mit einem der Anschlüsse 600a", 600b", 600c verbunden. Das erste Kompartiment 510a ist über das erste Leitungsmittel 550a" fluidleitend mit dem ersten Anschluss 600a" verbunden, das zweite Kompartiment 510b (nicht gezeigt in Figur 8, siehe beispielsweise Figur 9 oder 10) ist über das zweite Leitungsmittel 550b" fluidleitend mit dem zweiten Anschluss 600b'' verbunden und das dritte Kompartiment 510c (nicht gezeigt in Figur 8, siehe beispielsweise Figur 9 oder 10) ist über das dritte Leitungsmittel 550c fluidleitend mit dem dritten Anschluss 600c verbunden. Dazu weist jedes Kompartiment 510a, 510b, 510c jeweils eine Kompartimentdurchführung 515, welche in das jeweilige Leitungsmittel 550a", 550b", 550c mündet und so eine fluidleitende Verbindung herstellt, auf (lediglich die Kompartimentdurchführung 515 des ersten Kompartiments 510a ist sichtbar).

**Figur 9** zeigt einen Ausschnitt des Systems 700" aus Figur 8 umfassend das Reservoir 500" in einer Aufsicht auf eine Längsachse des Systems 700". Das erste Kompartiment 510a ist vom zweiten Kompartiment 510b und vom dritten Kompartiment 510c jeweils durch eine Zwischenwand 520 abgetrennt. Die einzelnen Kompartimente 510a, 510b, 510c sind jeweils durch eine der Kompartimentdurchführungen 515 fluidleitend mit den jeweiligen Leitungsmitteln 550a", 550b", 550c" verbunden.

**Figur 10** zeigt das in Kompartimente 510a, 510b, 510c unterteilte Reservoir 500" des Systems 700" aus den Figuren 8 und 9 in einer perspektivischen Seitenansicht. Die zwei die Kompartimente 510a, 510b, 510c trennenden Zwischenwände 520 weisen jeweils eine Höhe 521 (lediglich für die das erste Kompartiment 510a und das zweite Kompartiment 510b trennende Zwischenwand 521 eingezeichnet) auf, welche die Volumina der einzelnen Kompartimente 510a, 510b, 510c mitbestimmt. Die Volumina der einzelnen Kompartimente 510a, 510b, 510c sind im Wesentlichen gleich groß, so dass in jedem Kompartiment 510a, 510b, 510c die gleiche Menge an Flüssigkeitskomponente 350" (nicht eingezeichnet) lagerbar ist. In der gezeigten Ausführungsform des Reservoirs 500" ist die Höhe 521 der Zwischenwände 520 nicht über die gesamte Erstreckung der jeweiligen Zwischenwände 520 konstant. In weiteren, nicht gezeigten Ausführungsformen ist die Höhe 521 der Zwischenwände 520 über die gesamte Erstreckung konstant. Die Kompartimente 510a, 510b, 510c weisen jeweils eine fluidleitend geöffnete, der Aufnahme 110'' der Figur 8 zugewandte, obere Kompartimentseite 511a, 511b, 511c auf, durch welche die Flüssigkeitskomponente 350" nach einem Öffnen des Behälters 300" der Figur 8 einfließen kann.

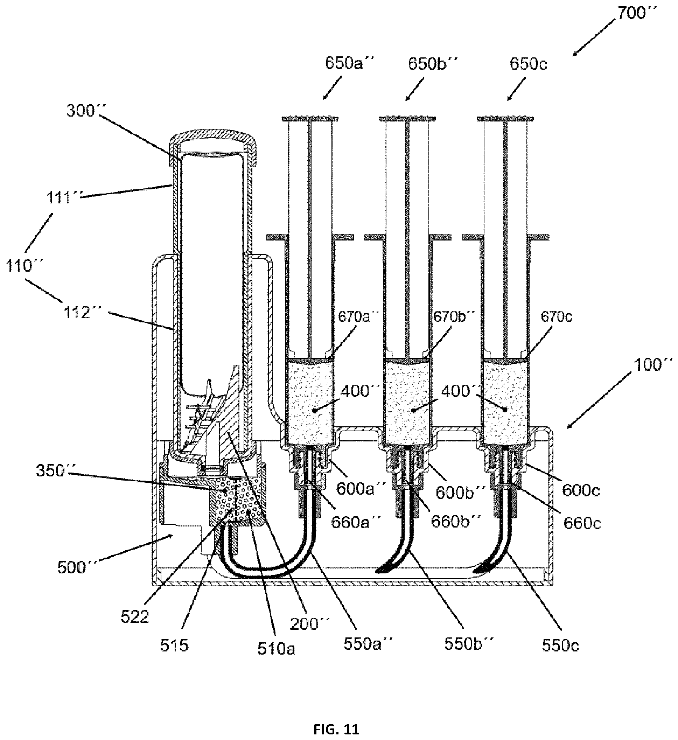

**Figur 11** zeigt das System 700" aus den Figuren 8 bis 10, wobei im Vergleich zu Figur 8 der hintere Aufnahmeabschnitt 111' nach Entfernen der Transportsicherung 113" der Figur 8 abschnittsweise in den vorderen Aufnahmeabschnitt 112' eingeschoben wurde, wodurch der Behälter 300" gegen das Öffnungsmittel 200" geschoben und dadurch fluidleitend geöffnet wurde. Die Flüssigkeitskomponente 350" ist aus dem Behälter 300" in das Reservoir 500", insbesondere durch die oberen Kompartimentseiten 511a, 511b, 511c der Figur 10, geflossen und hat die Kompartimente 510a, 510b, 510c gefüllt (lediglich gezeigt für das erste Kompartiment 510a). Die Menge an Flüssigkeitskomponente 350'' ist dabei so gewählt, dass die Kompartimente 510a, 510b, 510c dabei eine Füllhöhe 522 aufweisen, welche die Höhe 521 (vgl. Figur 10) der Zwischenwände 520 zumindest abschnittsweise überragt. Die Kompartimente 510a, 510b, 510c weisen dadurch einen Überstand an Flüssigkeitskomponente 350" auf, welcher sich über die oberen Kompartimentseiten 511a, 511b, 511c erstreckt. Der Überstand hat ein gleichmäßiges Befüllen aller Kompartimente 510a, 510b, 510c erlaubt, so dass es unerheblich war, ob die Flüssigkeitskomponente 350" aus dem Behälter 300" gleichmäßig in alle Kompartimente 510a, 510b, 510c oder ungleichmäßig, beispielsweise lediglich in das erste Kompartimente 510a, geflossen ist. Der Überstand an Flüssigkeitskomponente hat für eine Anpassung der Füllhöhe der einzelnen Kompartimente 510a, 510b, 510c gesorgt, da die Kompartimente 510a, 510b, 510c über die oberen Kompartimentseiten 511a, 511b, 511c fluidleitend miteinander verbunden sind.

Die Füllhöhe 522 der Kompartimente 510a, 510b, 510c ist lediglich 1 mm höher als die Höhe der Zwischenwände 520, so dass in jede der Spritzen 650a", 650b", 650c im Wesentlichen eine gleiche Menge an Flüssigkeitskomponente 350" förderbar ist.

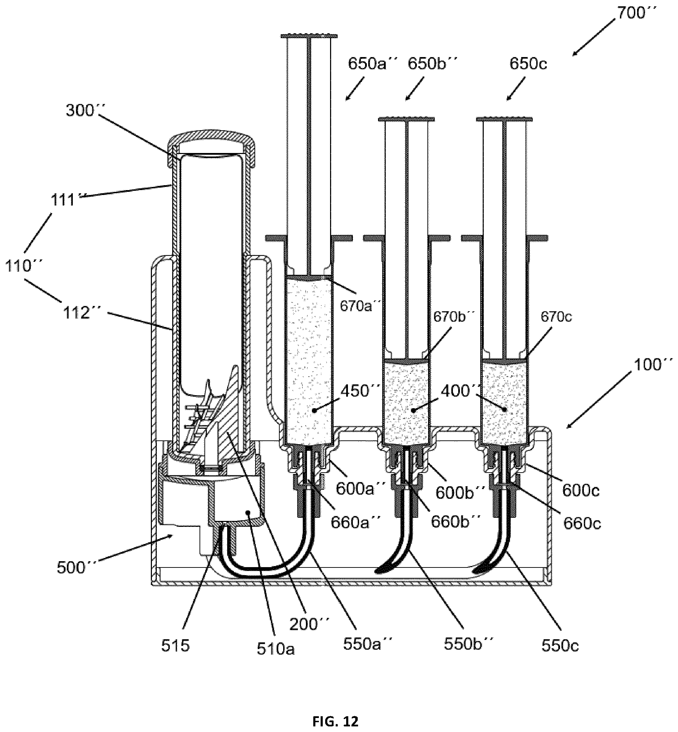

**Figur 12** zeigt das System 700" aus den Figuren 8 bis 11, wobei im Vergleich zu Figur 11 die Flüssigkeitskomponente 350" (nicht zu sehen) aus dem ersten Kompartiment 510a über das erste Leitungsmittel 550a" und den ersten Anschluss 600a" durch teilweises axiales Herausziehen des Spritzenkolbens 670a" der ersten Spritze 650a" in die erste Spritze 650a" gefördert wurde. In der ersten Spritze 650" hat sich dadurch aus den zwei Ausgangskomponenten ein Knochenzementteig 450" ausgebildet. Im zweiten Kompartiment 510b und im dritten Kompartiment 510c befindet sich noch Flüssigkeitskomponente 350" mit einer Füllhöhe 522, welche der niedrigsten Höhe 521 der Zwischenwände 520 entspricht (nicht gezeigt). Dies erlaubt ein zeitversetztes Bereitstellen des Knochenzementteigs 450'' in der zweiten Spritze 650b" und der dritten Spritze 650c im Vergleich zur Bereitstellung in der ersten Spritze 650a" mit den vorgenannten Vorteilen.

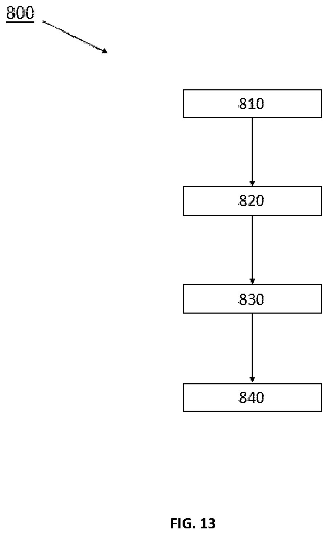

**Figur 13** zeigt ein Flussdiagramm eines Verfahrens 800 zum Bereitstellen eines Knochenzementteigs 350, 350', 350" mittels den Systemen 700, 700', 700" gemäß den Figuren 2 bis 6, 7 und 8 bis 12 umfassend die Schritte 810 bis 840.

In einem Schritt 810 wird der in der Aufnahme 110, 110', 110" lagernde Behälter 300, 300', 300" mittels das Öffnungsmittel 200, 200', 200" geöffnet. Vorzugsweise erfolgt das Öffnen 810 des Behälters 300, 300', 300" durch ein Einschieben des hinteren Aufnahmeabschnitts 11, 111'**,** 111'' in den vorderen Aufnahmeabschnitt 112, 112', 112", wodurch der Behälter 300, 300', 300", vorzugsweise in Form einer Glasampulle, gegen das Öffnungsmittel 200, 200', 200", vorzugsweise in Form einer Schräge, geschoben und dadurch geöffnet wird.

In einem Schritt 820 fließt die Flüssigkeitskomponente 350, 350', 350" nach dem Öffnen des Behälters 300, 300', 300" aus diesem heraus und in das Reservoir 500, 500', 500" hinein. In einer Ausführungsform des Verfahrens 800 fließt die Flüssigkeitskomponente 350" in ein in separate Kompartimente 510a, 510b, 510c unterteiltes Reservoir 500", was eine Vorportionierung der Flüssigkeitskomponente 350" innerhalb des Reservoirs 500" erlaubt und einem Anwender des Verfahrens 800 eine portionsweise und zeitversetzte Anmischung des Knochenzementteigs 450" erleichtert.

In einem Schritt 830 erfolgt ein Fördern eines ersten Teils der Flüssigkeitskomponente 350, 350', 350" aus dem Reservoir in die erste Spritze 650a, 650a', 650a".

In einer Ausführungsform erfolgt das Fördern 830 aus dem ersten Kompartiment 510a in die erste Spritze 650a, 650a', 650a".

In der ersten Spritze 650a, 650a', 650a" kann nach diesem Schritt 830 durch Mischung der zwei Ausgangskomponenten eine erste Portion des Knochenzementteigs 450, 450', 450" bereitgestellt werden. Vorzugsweise ist die erste Spritze 650a, 650a', 650a" mischeinrichtungsfrei ausgestaltet und die Bereitstellung kann ohne mechanische Einwirkung, sondern beispielsweise durch Schütteln der ersten Spritze 650a, 650a', 650a", durchgeführt werden. Nach dem Bereitstellen des Knochenzementteigs 450, 450', 450" in der ersten Spritze 650a, 650a', 650a" beginnt die Verarbeitungszeit des Knochenzementteigs 450, 450', 450". Der Knochenzementteigs 450, 450', 450" wird vorzugsweise innerhalb dieser Verarbeitungszeit verwendet.

Zeitversetzt, beispielsweise nach Ablauf der Verarbeitungszeit des Knochenzementteigs 450, 450', 450" in der ersten Spritze 650a, 650a', 650a" oder nach einem Aufbrauchen des Knochenzementteigs 450, 450', 450" in der ersten Spritze 650a, 650a', 650a", und unabhängig von Schritt 830 erfolgt in einem Schritt 840 ein Fördern eines zweiten Teils der Flüssigkeitskomponente 350, 350', 350" aus dem Reservoir in die zweite Spritze 650b, 650b', 650b".

In einer Ausführungsform erfolgt das Fördern 840 aus dem zweiten Kompartiment 510b in die zweite Spritze 650a, 650a', 650a".

In der zweiten Spritze 650b, 650b', 650b" kann nach diesem Schritt 840 durch Mischung der zwei Ausgangskomponenten eine zweite Portion des Knochenzementteigs 450, 450', 450" bereitgestellt werden. Vorzugsweise ist die zweite Spritze 650b, 650b', 650b" mischeinrichtungsfrei ausgestaltet und die Bereitstellung kann ohne mechanische Einwirkung, sondern beispielsweise durch Schütteln der zweiten Spritze 650b, 650b', 650b", durchgeführt werden. Nach dem Bereitstellen des Knochenzementteigs 450, 450', 450" in der zweiten Spritze 650b, 650b', 650b" beginnt die Verarbeitungszeit des Knochenzementteigs 450, 450', 450". Der Knochenzementteigs 450, 450', 450" wird vorzugsweise innerhalb dieser Verarbeitungszeit verwendet.

Das Verfahren 800 erlaubt eine portionsweise Bereitstellung des Knochenzementteigs 450, 450', 450", was eine Vereinfachung für einen Anwender des Verfahrens darstellt. Insbesondere verringert sich der Zeitdruck zur Durchführung des Verfahrens 800 aufgrund der spezifischen Verarbeitungszeit des verwendeten Knochenzementteigs 450, 450', 450" für den Anwender. Dies erlaubt eine breitere Auswahl unterschiedlicher Zusammensetzungen des Knochenzementteigs 450, 450', 450". Zudem ermöglicht das Verfahren einen ressourcenschonenderen Umgang mit einem einzigen Behälter 300, 300', 300" der Flüssigkeitskomponente 350, 350'. 350".

### Bezugszeichen

- 100, 100', 100": Vorrichtung
- 110, 110', 110": Aufnahme
- 111, 111'**,** 111": hinterer Aufnahmeabschnitt
- 112, 112', 112": vorderer Aufnahmeabschnitt
- 113, 113": Transportsicherung
- 200, 200', 200": Öffnungsmittel
- 300, 300', 300": Behälter
- 310: Glasampullenkopf
- 320: Glasampullenhals
- 330: Glasampullenkörper
- 350, 350', 350": Flüssigkeitskomponente
- 400, 400', 400": Pulverkomponente
- 450, 450', 450": Knochenzementteig
- 500, 500', 500": Reservoir
- 505: Reservoirdurchführung
- 510a: erstes Kompartiment
- 510b: zweites Kompartiment
- 510c: drittes Kompartiment
- 511a, 511b, 511c: obere Kompartimentseite
- 515: Kompartimentdurchführung
- 520: Zwischenwand
- 521: Höhe der Zwischenwand
- 522: Füllhöhe
- 550a, 550a'. 550a": erstes Leitungsmittel
- 550b, 550b', 550b": zweites Leitungsmittel
- 550c: drittes Leitungsmittel
- 600a, 600a', 600a": erster Anschluss
- 600b, 600b', 600b": zweiter Anschluss
- 600c: dritter Anschluss
- 610a, 610b: Filtereinheit
- 650a, 650a', 650a": erste Spritze
- 650b, 650b', 650b": zweite Spritze
- 650c: dritte Spritze
- 660a, 660b: Filter
- 660a", 660b", 660c 670a, 670b,: Spritzenkolben
- 670a', 670b' 670a", 670b'', 670c 700, 700', 700": System
- 800: Verfahren zum Bereitstellen eines Knochenzementteigs
- 810: Öffnen
- 820: Fließen
- 830: erstes Fördern
- 840: zweites Fördern

## Patentansprüche

1. Vorrichtung (100") zum Bereitstellen einer Flüssigkeitskomponente (350") als einer ersten Ausgangskomponente eines Knochenzementteigs (450") aus zwei Ausgangskomponenten, umfassend
eine Aufnahme (110"), in welcher ein Behälter (300") enthaltend die Flüssigkeitskomponente (350") lagerbar ist,
ein Öffnungsmittel (200") zum Öffnen des Behälters (300"),
ein mit der Aufnahme (110") fluidleitend verbundenes Reservoir (500") zur Aufnahme der Flüssigkeitskomponente (350") aus dem Behälter (300"),
eine Vielzahl an Anschlüssen (600a", 600b", 600c) zum fluidleitenden Verbinden der Vorrichtung (100") mit einer Spritze (650a", 650b", 650c), in welcher eine Pulverkomponente (400") als eine zweite Ausgangskomponente des Knochenzementteigs (450") lagerbar ist, und
eine Vielzahl an Leitungsmitteln (550a", 550b'', 550c), wobei jeweils eines der Leitungsmittel (550a", 550b", 550c) aus der Vielzahl an Leitungsmitteln (550a", 550b", 550c) das Reservoir (500") und einen der Anschlüsse (600a'', 600b", 600c) aus der Vielzahl der Anschlüsse (600a", 600b'', 600c) fluidleitend miteinander verbindet, wobei das Reservoir (500") in eine Vielzahl an Kompartimenten (510a, 510b, 510c) unterteilt ist und wobei jeweils eines der Kompartimente (510a, 510b, 510c) über eines der Leitungsmittel (550a", 550b", 550c) mit einem der Anschlüsse (600a", 600b", 600c) fluidleitend verbunden ist,
**dadurch gekennzeichnet, dass**
das Reservoir (500'') mittels mindestens einer Zwischenwand (520) in die Kompartimente (510a, 510b, 510c) unterteilt ist.

2. Vorrichtung (100") nach Anspruch 1, wobei die Kompartimente (510a, 510b, 510c) jeweils ein im Wesentlichen gleiches Volumen aufweisen.

3. Vorrichtung (100") nach einem der vorhergehenden Ansprüche, wobei die Kompartimente (510a, 510b, 510c) jeweils an einer der Aufnahme (110") zugewandten oberen Kompartimentseite (511a, 511b, 511c) fluidleitend geöffnet sind, so dass die Kompartimente (510a, 510b, 510c) über die oberen Kompartimentseiten (511a, 511b, 511c) fluidleitend miteinander verbunden sind.

4. Vorrichtung (100") nach einem der vorhergehenden Ansprüche, wobei das mindestens eine Leitungsmittel (550a", 550b", 550c) ein Schlauch ist.

5. Vorrichtung (100") nach Anspruch 4, wobei der Schlauch einen Innendurchmesser in einem Bereich von 0,5 mm bis 3 mm aufweist.

6. System (700") zum Bereitstellen eines Knochenzementteigs (450") aus zwei Ausgangskomponenten, umfassend eine Vorrichtung (100") gemäß einem der Ansprüche 1 bis 5, wobei in der Aufnahme (110") ein Behälter (300") enthaltend eine Flüssigkeitskomponente (350") als erste Ausgangskomponente lagert und umfassend eine Vielzahl an Spritzen (", 650b", 650c) jeweils enthaltend eine Pulverkomponente (400") als zweite Ausgangskomponente.

7. System (700") nach Anspruch 6, wobei die Vorrichtung (100") eine Vielzahl an Anschlüssen (600a", 600b'', 600c) aufweist und die Anzahl der Anschlüsse (600a", 600b", 600c) der Anzahl der Spritzen (650a", 650b", 650c) entspricht.

8. System (700") nach Anspruch 7, wobei die Spritzen (", 650b", 650c) reversibel fluidleitend mit den Anschlüssen (600a", 600b'', 600c) verbunden sind.

9. System (700") nach einem der Ansprüche 6 bis 8, wobei die Vorrichtung (100") eine Vorrichtung (100") nach einem der Ansprüche 3 bis 5 ist, wobei im Behälter (300") eine derartige Menge an Flüssigkeitskomponente (350") lagert, dass nach einem Öffnen des Behälters (300") alle Kompartimente (510a, 510b, 510c) des Reservoirs (500") mit der Flüssigkeitskomponente (350") gefüllt werden können und dabei eine Füllhöhe (522) aufweisen, welche eine Höhe (521) der die Kompartimente (510a, 510b, 510c) trennenden mindestens einen Zwischenwand (520) zumindest abschnittsweise überragt.

10. System (700") nach Anspruch 9, wobei die Füllhöhe (522) der Kompartimente (510a, 510b, 510c) zumindest abschnittsweise die Höhe (521) der Zwischenwand um maximal 1 mm überragt.

11. System (700") nach einem der Ansprüche 6 bis 10, wobei in den Spritzen (650a", 650b", 650c) eine im Wesentlichen gleiche Menge der Pulverkomponente (400") gelagert ist.

12. Verfahren (800) zum Bereitstellen eines Knochenzementteigs (450, 450', 450") aus zwei Ausgangskomponenten mittels eines Systems (700, 700', 700"), umfassend eine Vorrichtung (100, 100', 100) umfassend eine Aufnahme (110, 110', 110"), in welcher ein Behälter (300, 300', 300") enthaltend die Flüssigkeitskomponente (350, 350', 350") lagerbar ist, ein Öffnungsmittel (200, 200', 200") zum Öffnen des Behälters (300, 300', 300''), ein mit der Aufnahme (110, 110', 110") fluidleitend verbundenes Reservoir (500, 500', 500") zur Aufnahme der Flüssigkeitskomponente (350, 350', 350'') aus dem Behälter (300, 300', 300''), mindestens einen Anschluss (600a, 600b, 600a', 600b', 600a", 600b", 600c) zum fluidleitenden Verbinden der Vorrichtung (100, 100', 100") mit einer Spritze (650a, 650b, 650a', 650b', 650a", 650b", 650c), in welcher eine Pulverkomponente (400, 400', 400") als eine zweite Ausgangskomponente des Knochenzementteigs (450, 450', 450") lagerbar ist, und mindestens ein Leitungsmittel (550a, 550b, 550a', 550b', 550a", 550b", 550c), welches das Reservoir (500, 500', 500") und den mindestens einen Anschluss (600a, 600b, 600a', 600b', 600a", 600b", 600c) fluidleitend miteinander verbindet, wobei in der Aufnahme (110, 110', 110") ein Behälter (300, 300', 300") enthaltend eine Flüssigkeitskomponente (350, 350', 350") als erste Ausgangskomponente lagert und umfassend eine Vielzahl an Spritzen (650a, 650b, 650a', 650b', 650a", 650b", 650c) jeweils enthaltend eine Pulverkomponente (400, 400', 400") als zweite Ausgangskomponente, wobei die Vielzahl an Spritzen (650a, 650b, 650a', 650b', 650a", 650b", 650c) zumindest eine erste Spritze (650a, 650a', 650a") und eine zweite Spritze (650b, 650b', 650b'') umfasst, aufweisend die folgenden Schritte:
a. Öffnen (810) des Behälters (300, 300', 300") mittels des Öffnungsmittels (200, 200', 200"),
b. Fließen (820) der Flüssigkeitskomponente (350, 350', 350") aus dem geöffneten Behälter (300, 300', 300") in das Reservoir (500, 500', 500"),
c. Fördern (830) eines ersten Teils der Flüssigkeitskomponente (350, 350', 350") aus dem Reservoir (500, 500', 500") in die erste Spritze (650a, 650a', 650a"),
d. Fördern (840) eines zweiten Teils der Flüssigkeitskomponente (350, 350', 350") aus dem Reservoir (500, 500', 500") in die zweite Spritze (650b, 650b', 650b").

13. Verfahren (800) nach Anspruch 12, wobei das System (700, 700', 700") einen ersten Anschluss (600a, 600a', 600a") und ein erstes Leitungsmittel (550a, 550a', 550a") zum fluidleitenden Verbinden des ersten Anschlusses (600a, 600a', 600a") mit dem Reservoir (500, 500', 500") sowie einen zweiten Anschluss (600b, 600b', 600b") und ein zweites Leitungsmittel (550b, 550b', 550b") zum fluidleitenden Verbinden des zweiten Anschlusses (600b, 600b', 600b") mit dem Reservoir (500, 500', 500") aufweist, wobei das Fördern (830) des ersten Teils der Flüssigkeitskomponente (350, 350', 350") aus dem Reservoir (500, 500', 500") in die erste Spritze (650a, 650a', 650a") über den ersten Anschluss (600a, 600a', 600a") und das Fördern (840) des zweiten Teils der Flüssigkeitskomponente (350, 350', 350'') aus dem Reservoir (500, 500', 500") in die zweite Spritze (650b, 650b', 650b") über den zweiten Anschluss (600b, 600b', 600b") erfolgt.

14. Verfahren (800) nach Anspruch 13, wobei das Reservoir (500") ein erstes Kompartiment (510a) und ein zweites Kompartiment (510b) aufweist, wobei nach dem Öffnen des Behälters (300") der erste Teil der Flüssigkeitskomponente (350") in das erste Kompartiment (510a) und der zweite Teil der Flüssigkeitskomponente (350") in das zweite Kompartiment (510b) fließt, wobei das erste Kompartiment (510a) über das erste Leitungsmittel (550a") mit dem ersten Anschluss (600a") und das zweite Kompartiment (510b) über das zweite Leitungsmittel (550b") mit dem zweiten Anschluss (600b") fluidleitend verbunden ist und das Fördern (830) des ersten Teils der Flüssigkeitskomponente (350)" aus dem ersten Kompartiment (510a) in die erste Spritze (650a") und das Fördern (840) des zweiten Teils der Flüssigkeitskomponente (350") aus dem zweiten Kompartiment (510b) in die zweite Spritze (650b") erfolgt.

## Claims

1. A device (100") for providing a liquid component (350") as a first starting component of a bone cement paste (450") from two starting components, comprising a receptacle (110") in which a container (300") containing the liquid component (350") can be stored,
an opening means (200") for opening the container (300"),
a reservoir (500") fluidically connected to the receptacle (110") for receiving the liquid component (350") from the container (300"),
a plurality of ports (600a", 600b", 600c) for fluidically connecting the device (100") to a syringe (650a", 650b", 650c) in which a powder component (400") can be stored as a second starting component of the bone cement paste (450"), and
a plurality of conduits (550a", 550b", 550c), each of the conduits (550a", 550b", 550c) from the plurality of conduits (550a", 550b", 550c) fluidically connecting the reservoir (500") and one of the ports (600a", 600b", 600c) from the plurality of ports (600a", 600b", 600c) to one another, the reservoir (500") being divided into a plurality of compartments (510a, 510b, 510c) and each of the compartments (510a, 510b, 510c) being fluidically connected to one of the ports (600a", 600b", 600c) via one of the conduits (550a", 550b", 550c),
**characterized in that**
the reservoir (500") is divided into the compartments (510a, 510b, 510c) by means of at least one partition wall (520).

2. The device (100") according to claim 1, wherein the compartments (510a, 510b, 510c) each have a substantially equal volume.

3. The device (100") according to either of the preceding claims, wherein the compartments (510a, 510b, 510c) are each fluidically open at one of the upper compartment sides (511a, 511b, 511c) facing the receptacle (110"), so that the compartments (510a, 510b, 510c) are fluidically connected to one another via the upper compartment sides (511a, 511b, 511c).

4. The device (100") according to any of the preceding claims, wherein the at least one conduit (550a", 550b", 550c) is a tube.

5. The device (100") according to claim 4, wherein the tube has an inner diameter in a range of 0.5 mm to 3 mm.

6. A system (700") for providing a bone cement paste (450") from two starting components, comprising a device (100") according to any of claims 1 to 5, wherein a container (300") containing a liquid component (350") as the first starting component stores in the receptacle (110"), and comprising a plurality of syringes (", 650b", 650c) each containing a powder component (400") as the second starting component.

7. The system (700") according to claim 6, wherein the device (100") comprises a plurality of ports (600a", 600b", 600c) and the number of ports (600a", 600b", 600c) corresponds to the number of syringes (650a", 650b", 650c).

8. The system (700") according to claim 7, wherein the syringes (", 650b", 650c) are reversibly fluidically connected to the ports (600a", 600b", 600c).

9. The system (700") according to any of claims 6 to 8, wherein the device (100") is a device (100") according to any of claims 3 to 5, wherein such an amount of liquid component (350") is stored in the container (300") that, after the container (300") is opened, all compartments (510a, 510b, 510c) of the reservoir (500") can be filled with the liquid component (350") and have a fill level (522) which at least partially exceeds a height (521) of the at least one partition wall (520) separating the compartments (510a, 510b, 510c).

10. The system (700") according to claim 9, wherein the fill level (522) of the compartments (510a, 510b, 510c) at least partially exceeds the height (521) of the partition wall by a maximum of 1 mm.

11. The system (700") according to any of claims 6 to 10, wherein a substantially equal amount of the powder component (400") is stored in the syringes (650a", 650b", 650c).

12. A method (800) for providing a bone cement paste (450, 450', 450") from two starting components by means of a system (700, 700', 700") comprising a device (100, 100', 100) comprising a receptacle (110, 110', 110") in which a container (300, 300', 300") containing the liquid component (350, 350', 350") can be stored, an opening means (200, 200', 200") for opening the container (300, 300', 300"), a reservoir (500, 500', 500") fluidically connected to the receptacle (110, 110', 110") for receiving the liquid component (350, 350', 350") from the container (300, 300', 300"), at least one port (600a, 600b, 600a', 600b', 600a", 600b", 600c) for fluidically connecting the device (100, 100', 100") to a syringe (650a, 650b, 650a', 650b', 650a", 650b", 650c) in which a powder component (400, 400', 400") can be stored as a second starting component of the bone cement paste (450, 450', 450"), and at least one conduit (550a, 550b, 550a', 550b', 550a", 550b", 550c), which fluidically connects the reservoir (500, 500', 500") and the at least one port (600a, 600b, 600a', 600b', 600a", 600b", 600c) to one another, wherein a container (300, 300', 300") containing a liquid component (350, 350', 350") as the first starting component is stored in the receptacle (110, 110', 110") and comprising a plurality of syringes (650a, 650b, 650a', 650b', 650a", 650b", 650c) each containing a powder component (400, 400', 400") as the second starting component, wherein the plurality of syringes (650a, 650b, 650a', 650b', 650a", 650b", 650c) comprises at least a first syringe (650a, 650a', 650a") and a second syringe (650b, 650b', 650b"), comprising the following steps:
a. opening (810) of the container (300, 300', 300") by means of the opening means (200, 200', 200"),
b. flowing (820) of the liquid component (350, 350', 350") from the open container (300, 300', 300") into the reservoir (500, 500', 500"),
c. conveying (830) of a first part of the liquid component (350, 350', 350") from the reservoir (500, 500', 500") into the first syringe (650a, 650a', 650a"),
d. conveying (840) of a second part of the liquid component (350, 350', 350") from the reservoir (500, 500', 500") into the second syringe (650b, 650b', 650b").

13. The method (800) according to claim 12, wherein the system (700, 700', 700") comprises a first port (600a, 600a', 600a") and a first conduit (550a, 550a', 550a") for fluidically connecting the first port (600a, 600a', 600a") to the reservoir (500, 500', 500"), and a second port (600b, 600b', 600b") and a second conduit (550b, 550b', 550b") for fluidically connecting the second port (600b, 600b', 600b") to the reservoir (500, 500', 500"), wherein the conveying (830) of the first part of the liquid component (350, 350', 350") from the reservoir (500, 500', 500") into the first syringe (650a, 650a', 650a") takes place via the first port (600a, 600a', 600a") and the conveying (840) of the second part of the liquid component (350, 350', 350") from the reservoir (500, 500', 500") into the second syringe (650b, 650b', 650b") takes place via the second port (600b, 600b', 600b").

14. The method (800) according to claim 13, wherein the reservoir (500") comprises a first compartment (510a) and a second compartment (510b), wherein, after the container (300") is opened, the first part of the liquid component (350") flows into the first compartment (510a) and the second part of the liquid component (350") flows into the second compartment (510b), wherein the first compartment (510a) is fluidically connected to the first port (600a") via the first conduit (550a") and the second compartment (510b) is fluidically connected to the second port (600b") via the second conduit (550b") and the conveying (830) of the first part of the liquid component (350)" from the first compartment (510a) into the first syringe (650a") takes place and the conveying (840) of the second part of the liquid component (350") from the second compartment (510b) into the second syringe (650b") takes place.

## Revendications

1. Dispositif (100") pour la fourniture d'un composant liquide (350") en tant que premier composant de départ d'une pâte de ciment osseux (450") à partir de deux composants de départ, comprenant
un logement (110") dans lequel un récipient (300") contenant le composant liquide (350") peut être stocké,
un moyen d'ouverture (200") pour l'ouverture du récipient (300"),
un réservoir (500") relié d'une manière conductrice de fluide au logement (110") et permettant de recevoir le composant liquide (350") provenant du récipient (300"),
une pluralité de raccords (600a", 600b", 600c) pour la liaison d'une manière conductrice de fluide du dispositif (100") à une seringue (650a", 650b", 650c) dans laquelle un composant en poudre (400") peut être stocké en tant que second composant de départ de la pâte de ciment osseux (450"), et
une pluralité de moyens de conduite (550a", 550b", 550c), dans lequel respectivement l'un des moyens de conduite (550a", 550b", 550c) de la pluralité de moyens de conduite (550a", 550b", 550c) relie d'une manière conductrice de fluide le réservoir (500") et l'un des raccords (600a", 600b", 600c) de la pluralité de raccords (600a", 600b", 600c) l'un à l'autre, dans lequel le réservoir (500") est divisé en une pluralité de compartiments (510a, 510b, 510c) et dans lequel respectivement l'un des compartiments (510a, 510b, 510c) est relié d'une manière conductrice de fluide à l'un des raccords (600a", 600b", 600c) par l'intermédiaire de l'un des moyens de conduite (550a", 550b", 550c),
**caractérisé en ce que**
le réservoir (500") est divisé en compartiments (510a, 510b, 510c) au moyen d'au moins une paroi intermédiaire (520).

2. Dispositif (100") selon la revendication 1, dans lequel les compartiments (510a, 510b, 510c) présentent respectivement un volume sensiblement identique.

3. Dispositif (100") selon l'une des revendications précédentes, dans lequel les compartiments (510a, 510b, 510c) sont respectivement ouverts d'une manière conductrice de fluide sur un côté de compartiment supérieur (511a, 511b, 511c) tourné vers le logement (110"), de sorte que les compartiments (510a, 510b, 510c) sont reliés d'une manière conductrice de fluide les uns aux autres par l'intermédiaire des côtés de compartiment supérieurs (511a, 511b, 511c).

4. Dispositif (100") selon l'une des revendications précédentes, dans lequel l'au moins un moyen de conduite (550a", 550b", 550c) est un tuyau.

5. Dispositif (100") selon la revendication 4, dans lequel le tuyau présente un diamètre intérieur compris dans une plage allant de 0,5 mm à 3 mm.

6. Système (700") pour la fourniture d'une pâte de ciment osseux (450") à partir de deux composants de départ, comprenant un dispositif (100") conformément à l'une des revendications 1 à 5, dans lequel un récipient (300") contenant un composant liquide (350") comme premier composant de départ est stocké dans le logement (110") et comprenant une pluralité de seringues (", 650b", 650c) contenant respectivement un composant en poudre (400") comme second composant de départ.

7. Système (700") selon la revendication 6, dans lequel le dispositif (100") présente une pluralité de raccords (600a", 600b", 600c) et le nombre de raccords (600a", 600b", 600c) correspond au nombre de seringues (650a", 650b", 650c).

8. Système (700") selon la revendication 7, dans lequel les seringues (", 650b", 650c) sont reliées de manière réversible et d'une manière conductrice de fluide aux raccords (600a", 600b", 600c).

9. Système (700") selon l'une des revendications 6 à 8, dans lequel le dispositif (100") est un dispositif (100") selon l'une des revendications 3 à 5, dans lequel le récipient (300") stocke une quantité de composant liquide (350") telle qu'après une ouverture du récipient (300"), tous les compartiments (510a, 510b, 510c) du réservoir (500") peuvent être remplis du composant liquide (350") et présentent alors une hauteur de remplissage (522) qui dépasse, au moins dans certaines sections, une hauteur (521) de l'au moins une paroi intermédiaire (520) séparant les compartiments (510a, 510b, 510c).

10. Système (700") selon la revendication 9, dans lequel la hauteur de remplissage (522) des compartiments (510a, 510b, 510c) dépasse, au moins dans certaines sections, la hauteur (521) de la paroi intermédiaire de 1 mm au maximum.

11. Système (700") selon l'une des revendications 6 à 10, dans lequel une quantité sensiblement égale du composant en poudre (400") est stockée dans les seringues (650a", 650b", 650c).

12. Procédé (800) pour la fourniture d'une pâte de ciment osseux (450, 450', 450") à partir de deux composants de départ au moyen d'un système (700, 700', 700"), comprenant un dispositif (100, 100', 100) comprenant un logement (110, 110', 110") dans lequel un récipient (300, 300', 300") contenant le composant liquide (350, 350', 350") peut être stocké, un moyen d'ouverture (200, 200', 200") pour l'ouverture du récipient (300, 300', 300"), un réservoir (500, 500', 500") relié d'une manière conductrice de fluide au logement (110, 110', 110") et permettant de recevoir le composant liquide (350, 350', 350") provenant du récipient (300, 300', 300"), au moins un raccord (600a, 600b, 600a', 600b', 600a", 600b", 600c) pour la liaison d'une manière conductrice de fluide du dispositif (100, 100', 100") à une seringue (650a, 650b, 650a', 650b", 650a", 650b", 650c) dans laquelle un composant en poudre (400, 400', 400") peut être stocké en tant que second composant de départ de la pâte de ciment osseux (450, 450', 450"), et au moins un moyen de conduite (550a, 550b, 550a', 550b', 550a", 550b", 550c) qui relie d'une manière conductrice de fluide le réservoir (500, 500', 500") et l'au moins un raccord (600a, 600b, 600a', 600b', 600a", 600b", 600c) l'un à l'autre, dans lequel un récipient (300, 300', 300") contenant un composant liquide (350, 350', 350") comme premier composant de départ est stocké dans le logement (110, 110', 110") et comprenant une pluralité de seringues (650a, 650b, 650a', 650b', 650a", 650b", 650c) contenant respectivement un composant en poudre (400, 400', 400") comme second composant de départ, dans lequel la pluralité de seringues (650a, 650b, 650a', 650b', 650a", 650b", 650c) comprennent au moins une première seringue (650a, 650a', 650a") et une seconde seringue (650b, 650b', 650b"), présentant les étapes suivantes :
a. ouverture (810) du récipient (300, 300', 300") à l'aide du moyen d'ouverture (200, 200', 200"),
b. écoulement (820) du composant liquide (350, 350', 350") provenant du récipient (300, 300', 300") ouvert dans le réservoir (500, 500', 500"),
c. transport (830) d'une première partie du composant liquide (350, 350', 350") depuis le réservoir (500, 500', 500") dans la première seringue (650a, 650a', 650a"),
d. transport (840) d'une seconde partie du composant liquide (350, 350', 350") depuis le réservoir (500, 500', 500") dans la seconde seringue (650b, 650b', 650b").

13. Procédé (800) selon la revendication 12, dans lequel le système (700, 700', 700") présente un premier raccord (600a, 600a', 600a") et un premier moyen de conduite (550a, 550a', 550a") pour la liaison d'une manière conductrice de fluide du premier raccord (600a, 600a', 600a") au réservoir (500, 500', 500") ainsi qu'un second raccord (600b, 600b', 600b") et un second moyen de conduite (550b, 550b', 550b") pour la liaison d'une manière conductrice de fluide du second raccord (600b, 600b', 600b") au réservoir (500, 500', 500"), dans lequel le transport (830) de la première partie du composant liquide (350, 350', 350") depuis le réservoir (500, 500', 500") dans la première seringue (650a, 650a', 650a") est effectué par l'intermédiaire du premier raccord (600a, 600a', 600a") et le transport (840) de la seconde partie du composant liquide (350, 350', 350") depuis le réservoir (500, 500', 500") dans la seconde seringue (650b, 650b', 650b") est effectué par l'intermédiaire du second raccord (600b, 600b', 600b").

14. Procédé (800) selon la revendication 13, dans lequel le réservoir (500") présente un premier compartiment (510a) et un second compartiment (510b), dans lequel, après l'ouverture du récipient (300"), la première partie du composant liquide (350") s'écoule dans le premier compartiment (510a) et la seconde partie du composant liquide (350") s'écoule dans le second compartiment (510b), dans lequel le premier compartiment (510a) est relié d'une manière conductrice de fluide au premier raccord (600a") par l'intermédiaire du premier moyen de conduite (550a") et le second compartiment (510b) est relié d'une manière conductrice de fluide au second raccord (600b") par l'intermédiaire du second moyen de conduite (550b"), et le transport (830) de la première partie du composant liquide (350") est effectué depuis le premier compartiment (510a) dans la première seringue (650a") et le transport (840) de la seconde partie du composant liquide (350") est effectué depuis le second compartiment (510b) dans la seconde seringue (650b").
